# EUROPEAN PATENT APPLICATION

(11) **EP 4 406 641 A1**
(43) Date of publication of application: **31.07.2024**
(21) Application number: 23382062.0
(22) Date of filing: 26.01.2023
(51) Int. Cl.: B01J 13/18, A01N 25/28, A23L 27/00, A23P 10/30, A61K 8/11, A61K 9/48, A61Q 5/00, A61Q 19/00, A61Q 19/10, B01J 13/20, C11D 3/50

(54) **BIODEGRADABLE MICROCAPSULES CONTAINING LOW LOG P FRAGRANCE**

(71) Applicant: International Flavors & Fragrances Inc., New York, NY 10019 (US)
(72) Inventor: The designation of the inventor has not yet been filed
(74) Representative: Barker Brettell LLP

(57) **Abstract**

This disclosure relates to a core-shell microcapsule slurry that includes (a) core-shell microcapsules having diameter of 1 to 100 microns, the core of the microcapsules contains an active material and the shell of the microcapsules contains a self-condensed polyisocyanate; (b) a dispersant containing denatured pea protein; and (c) a hydrocolloid containing gum Arabic; wherein the active material contains a low logP fragrance having a logP value ranging from 0.5 to 2.2, the amount of the low logP fragrance is from 2% to 18% by weight based on the weight of the active material, and the core-shell microcapsule slurry is white. This disclosure also relates to a method of producing such core-shell microcapsule slurry. This disclosure also relates to a consumer product containing such core-shell microcapsule slurry.

## Description

### FIELD OF THE DISCLOSURE

The present disclosure relates to a biodegradable core-shell microcapsule slurry composed of microcapsules. Particularly, the microcapsules have walls formed by self-polymerization of polyisocyanate in the presence of a denatured pea protein as dispersant. The microcapsules also have cores comprising low logP fragrance. Also disclosed are consumer products containing such core-shell microcapsule slurry and methods for producing such core-shell microcapsule slurry.

### BACKGROUND OF THE DISCLOSURE

Microcapsules are useful in a variety of applications where there is a need to deliver, apply, or release a fragrance or other active material in a time-delayed and controlled manner.

Conventional microcapsules each have a polymeric shell encapsulating an active material in a microcapsule core. The polymeric shell is typically formed *via* an interfacial polymerization reaction, namely, a polymerization that occurs at an interface between an aqueous phase and an oil phase. These microcapsules have been developed to provide good performance in various consumer products such as laundry detergents. See, *e.g*., US 7,491,687, US 6,045,835, US 2014/0287008, and WO 2015/023961. Polyurea microcapsules have been developed for delivering fragrances. Their preparation involves the polymerization reaction between wall-forming materials, *e.g*., a polyisocyanate and a polyamine. During the polymerization reaction, the polyisocyanate can react with many fragrance ingredients such as primary alcohols contained in a fragrance accord. The other wall-forming material polyamine is also reactive towards aldehyde fragrance ingredients. Primary alcohols and aldehydes are common ingredients in many fragrance accords. Such fragrances are not suitable to be encapsulated by conventional microcapsules. In addition, fragrance ingredients having a high-water solubility are also unsuitable for conventional encapsulation as these ingredients tend to stay in the aqueous phase instead of being encapsulated in the microcapsule oil core. Challenges remain in encapsulating fragrances and other active materials without losing reactive or water-soluble ingredients.

Methods to incorporate biodegradable polymers into microcapsule compositions have been described. For example, US 10,034,819 B2 and US 2019/0240124 A1 teach microcapsules with an inner shell and outer shell, wherein the outer shell is produced by complex coacervation of first polyelectrolyte such as gelatin and a second polyelectrolyte such as carboxymethyl cellulose, sodium carboxymethyl guar gum, xanthan gum and plant gums.

Similarly, EP 2588066 B1 describes a coacervated capsule prepared with a coating layer composed of a protein, and optionally a non-protein polymer.

Further, EP 2811846 B1 describes the use of protein aggregates as an interface layer around a hydrophobic substance.

EP 1855544 B8 teaches the use of the encapsulation of an active ingredient in a matrix composed of 0.5-95 wt% of anionic polysaccharides and 0.5-95 wt% of peptides having a molecular mass within the range of 0.3-12 kDa.

EP 3746217 A1 and WO 2020/195132 A1 describe the preparation of core-shell microcapsules by cross-linking a protein into the wall of the microcapsule.

US 10,166,196 B2 discloses an agglomeration of primary microcapsules composed of a primary shell and outer shell, wherein the outer shell is the primary shell and outer shell are products of a complex coacervation reaction of a first protein such as a pea or soy protein and a second polymer such as an agar, gellan gum, gum Arabic, casein, cereal prolamine, pectin, alginate, carrageenan, xanthan gum, canola protein, dilutan gum, locus bean gum, or welan gum.

As such, these existing solutions still have limitations and do not adequately teach how to overcome the above-mentioned problems. Accordingly, there is still a need to develop a microcapsule composition suitable for encapsulating active materials having ingredients that are sustainable and biodegradable.

### BRIEF SUMMARY OF THE DISCLOSURE

The present disclosure provides a core-shell microcapsule slurry. The core-shell microcapsule slurry comprises: (a) core-shell microcapsules having diameter of 1 to 100 microns, the core of the microcapsules comprises an active material and the shell of the microcapsules comprises a self-condensed polyisocyanate; (b) a dispersant comprising denatured pea protein; and (c) a hydrocolloid comprising gum Arabic; wherein the active material comprises a low logP fragrance having a logP value ranging from 0.5 to 2.2, the amount of the low logP fragrance is from 2% to 18% by weight based on the weight of the active material, and the core-shell microcapsule slurry is white.

### BRIEF DESCRIPTION OF THE FIGURES

Embodiments are illustrated in the accompanying figures to improve understanding of concepts as presented herein.

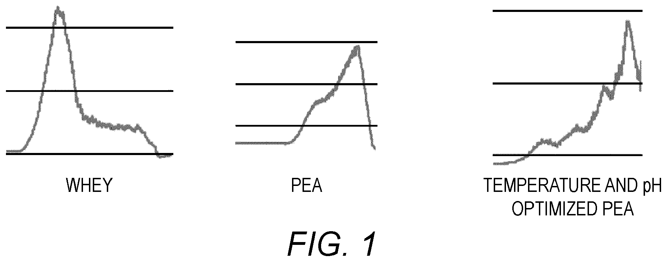

FIG. 1 shows the force curve generated in a capsule breaking experiment for capsules prepared with whey protein according to Example 7 of WO 2020/131875 A2 with the addition of citric acid prior to curing to achieve a cure pH of 5; pea protein according to Example 2 herein; and pea protein with optimized cure temperatures and pH as described in Example 3 herein. This analysis indicated that capsule wall properties could be modified by the protein select and, more importantly, by optimizing the curing profile and pH of the capsule formation reaction.

### DETAILED DESCRIPTION

The foregoing general description and the following detailed description are exemplary and explanatory only and are not restrictive of the invention, as defined in the appended claims. Other features and benefits of any one or more of the embodiments will be apparent from the following detailed description, and from the claims.

As used herein, the terms "comprise", "comprising", "include", "including," "have", "having", "contain", "containing" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, use of "a" or "an" are employed to describe elements and components described herein. This is done merely for convenience and to give a general sense of the scope of the invention. This description should be read to include one or at least one and the singular also includes the plural unless it is obvious that it is meant otherwise.

Unless otherwise defined, all technical and scientific terms used herein have the same meaning as commonly understood by one of ordinary skill in the art to which this invention belongs. In case of conflict, the present specification, including definitions, will control. Although methods and materials similar or equivalent to those described herein can be used in the practice or testing of embodiments of the present invention, suitable methods and materials are described below. In addition, the materials, methods, and examples are illustrative only and not intended to be limiting.

When an amount, concentration, or other value or parameter is given as either a range, preferred range or a list of upper preferable values and/or lower preferable values, this is to be understood as specifically disclosing all ranges formed from any pair of any upper range limit or preferred value and any lower range limit or preferred value, regardless of whether ranges are separately disclosed. Where a range of numerical values is recited herein, unless otherwise stated, the range is intended to include the endpoints thereof, and all integers and fractions within the range. For example, when a range of "1 to 10" is recited, the recited range should be construed as including ranges "1 to 8", "3 to 10", "2 to 7", "1.5 to 6", "3.4 to 7.8", "1 to 2 and 7-10", "2 to 4 and 6 to 9", "1 to 3.6 and 7.2 to 8.9", "1-5 and 10", "2 and 8 to 10", "1.5-4 and 8", and the like.

The present disclosure illustratively described herein suitably may be practiced in the absence of any element or elements, limitation or limitations that are not specifically disclosed herein. While compositions and methods are described herein in terms of "comprising" various components or steps, the compositions and methods also can "consist essentially of" or "consist of" the various components or steps, unless stated otherwise.

All parts, percentages and proportions referred to herein and in the claims are by weight unless otherwise indicated.

Before addressing details of embodiments described below, some terms are defined or clarified.

The term "elevated temperature", as used herein, means a temperature higher than the room temperature.

As used herein, the terms "capsule", "microcapsule" and "core-shell microcapsule" are used interchangeably and refer to a substantially spherical structure having a well-defined core and a well-defined envelope or wall or shell. The "core" comprises an active material or material submitted to microencapsulation. The terms "wall" and "shell" are used interchangeably to denote the structure formed by the microencapsulating polymer surrounding the active material core being microencapsulated.

The term "logP", as used herein, means the octanol/water partitioning coefficient (P) of a fragrance ingredient given in the form of its logarithm to the base 10, logP. The octanol/water partitioning coefficient of a fragrance ingredient is the ratio between its equilibrium concentrations in octanol and in water. The logP values of many fragrance ingredients have been reported, for example, in the Pomona92 database available from Daylight Chemical Information Systems, Inc. (Daylight CIS) in Irvine, California, USA.

The term "self-condensed polyisocyanate", as used herein, means the polyurea formed by the self-polymerization of polyisocyanate in the presence of water. A person skilled in the art appreciates that isocyanate can react with water to form amine which can further react with isocyanate to form urea linkage. Accordingly, polyisocyanate can self-polymerize in the presence of water to form polyurea.

As used herein, the terms "g," "mg," and "µg" refer to "gram," "milligram," and "microgram," respectively. The terms "L" and "mL" refer to "liter" and "milliliter," respectively.

### Polyisocyanate

As used herein, the terms "polyfunctional isocyanate" and "polyisocyanate" can be used interchangeably and refer to a compound having two or more isocyanate (-NCO) groups. Polyisocyanates can be aromatic, aliphatic, linear, branched, or cyclic. In some embodiments, the polyisocyanate contains, on average, 2 to 4 isocyanate groups. In some embodiments, the polyisocyanate contains at least three isocyanate functional groups. In some embodiments, the polyisocyanate is water insoluble. In certain aspects, the polyisocyanate is an oligomeric polyisocyanate obtained from hexamethylene diisocyanate (HDI), which is a monomeric diisocyanate. In certain aspects, the polyisocyanate is an oligomeric polyisocyanate having a biuret, isocyanurate, allophanate, uretdione and/or oligomeric HDI structure. Exemplary polyisocyanates are sold under the tradenames TAKENATE^{®} (*e.g*., TAKENATE^{®} D-110N; Mitsui Chemicals), DESMODUR^{®} (Covestro), BAYHYDUR^{®} (Covestro), and LUPRANATE^{®} (BASF).

In some embodiments, the polyisocyanate is an aromatic polyisocyanate. Desirably, the aromatic polyisocyanate includes a phenyl, tolyl, xylyl, naphthyl or diphenyl moiety as the aromatic component. In some embodiments, the aromatic polyisocyanate is selected from the group consisting of polyisocyanurate of toluene diisocyanate, trimethylol propane-adduct of toluene diisocyanate, trimethylol propane-adduct of xylylene diisocyanate, and mixtures thereof.

In some embodiments, the aromatic polyisocyanate has the structural formula shown below, and includes structural isomers thereof 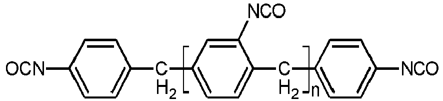 wherein n can vary from zero to a desired number (*e.g.,* 0-50, 0-20, 0-10, or 0-6). Preferably, the number of n is limited to less than 6. The polyisocyanate may also be a mixture of polyisocyanates where the value of n can vary from 0 to 6. In the case where the polyisocyanate is a mixture of various polyisocyanates, the average value of n preferably falls in between 0.5 and 1.5.

In some embodiments, the aromatic polyisocyanate has the structural formula shown below, and includes structural isomers thereof 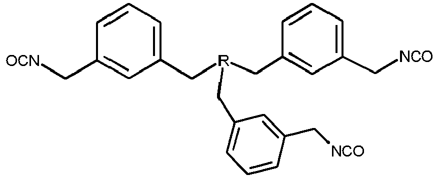 wherein R can be a C₁-C₁₀ alkyl, C₁-C₁₀ ester, or an isocyanurate. Representative polyisocyanates having this structure are sold under the trademarks TAKENATE^{®} D-110N (Mitsui), DESMODUR^{®} L75 (Covestro), and DESMODUR^{®} IL (Covestro).

Trimethylol propane-adduct of xylylene diisocyanate has the structural formula shown below:

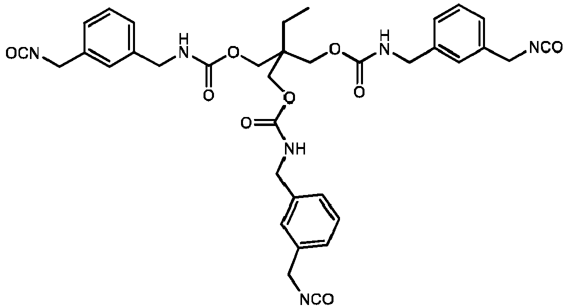

In some embodiments, the aromatic polyisocyanate is selected from the group consisting of 1,5-naphthylene diisocyanate, 4,4'-diphenylmethane diisocyanate (MDI), hydrogenated MDI (H12MDI), xylylene diisocyanate (XDI), tetramethylxylol diisocyanate (TMXDI), 4,4'-diphenyldimethylmethane diisocyanate, di- and tetraalkyldiphenylmethane diisocyanate, 4,4'-dibenzyl diisocyanate, 1,3-phenylene diisocyanate, 1,4-phenylene diisocyanate, the isomers of tolylene diisocyanate (TDI), 4,4'-diisocyanatophenylperfluoroethane, phthalic acid bisisocyanatoethyl ester, aromatic polyisocyanates with reactive halogen atoms, and mixtures thereof. In some embodiments, the aromatic polyisocyanate with reactive halogen atom is selected from the group consisting of 1-chloromethylphenyl 2,4-diisocyanate, 1-bromomethyl-phenyl 2,6-diisocyanate, 3,3-bischloromethyl ether 4,4'-diphenyldiisocyanate, and mixtures thereof.

In some embodiments, the polyisocyanate is an aliphatic polyisocyanate. In some embodiments, the aliphatic polyisocyanate is selected from the group consisting of trimer of hexamethylene diisocyanate, trimer of isophorone diisocyanate, biuret of hexamethylene diisocyanate, and mixtures thereof. In some embodiments, the aliphatic polyisocyanate is selected from the group consisting of 1-methyl-2,4-diisocyanatocyclohexane, 1,6-diisocyanato-2,2,4-trimethylhexane, 1,6-diisocyanato-2,4,4-trimethylhexane, 1-isocyanatomethyl-3-isocyanato-1,5,5-trimethylcyclohexane, chlorinated aliphatic diisocyanates, brominated aliphatic diisocyanates, phosphorus-containing aliphatic diisocyanates, tetramethoxybutane 1,4-diisocyanate, butane 1,4-diisocyanate, hexane 1,6-diisocyanate (HDI), dicyclohexylmethane diisocyanate, cyclohexane 1,4-diisocyanate, ethylene diisocyanate, and mixtures thereof. In some embodiments, the polyisocyanate comprises a sulfur-containing polyisocyanate which can be obtained, for example, by reacting hexamethylene diisocyanate with thiodiglycol or dihydroxydihexyl sulfide. In some embodiments, the polyisocyanate is an aliphatic diisocyanate selected from the group consisting of trimethylhexamethylene diisocyanate, 1,4-diisocyanatobutane, 1,2-diisocyanatododecane, dimer fatty acid diisocyanate, and mixtures thereof.

In some embodiments, the weight average molecular weight of the polyisocyanate ranges from 250 Da to 1000 Da, or from 275 Da to 500 Da. In some embodiments, the polyisocyanate used in the preparation of the shell of the microcapsule is a single polyisocyanate. In other embodiments, the polyisocyanate is a mixture of polyisocyanates. In some embodiments, the mixture of polyisocyanates includes an aliphatic polyisocyanate and an aromatic polyisocyanate. In some embodiments, the polyisocyanate is a mixture of a biuret of hexamethylene diisocyanate and a trimethylol propane-adduct of xylylene diisocyanate. In some embodiments, the polyisocyanate is an aliphatic polyisocyanate or a combination of aliphatic polyisocyanates, free of any aromatic polyisocyanate. In some embodiments, the polyisocyanate is trimethylol propane-adduct of xylylene diisocyanate, and the shell of the microcapsule comprises a self-condensed trimethylol propane-adduct of xylylene diisocyanate.

### Active Material

The core of the microcapsule comprises an active material encapsulated therein. Non-limiting examples include those described in WO 2016/049456. These active materials include fragrance, pro-fragrance, flavor, malodor counteractive agent, vitamin or derivative thereof, anti-inflammatory agent, anesthetic, analgesic, antimicrobial active, anti-viral agent, anti-infectious agent, anti-acne agent, skin lightening agent, insect repellent, animal repellent, vermin repellent, emollient, skin moisturizing agent, wrinkle control agent, UV protection agent, fabric softener active, hard surface cleaning active, skin or hair conditioning agent, flame retardant, antistatic agent, taste modulator, cell, probiotic, antioxidant, self-tanning agent, dihydroxyacetone, cooler, sensate, malodor reactive material, cosmetic active, agricultural active, pesticide, insecticide, herbicide, fungicide, or a combination thereof. Cosmetic actives include vitamins, sun filters and sunscreens, anti-aging agents, anti-wrinkle agents, antioxidants, lifting agents, firming agents, anti-spot agents, anti-redness agents, thinning agents, draining agents, moisturizers, soothing agents, scrubbing or exfoliating agents, mattifying agents, sebum regulating agents, skin-lightening actives, self-tanning actives, tanning accelerators, or a combination thereof. In some embodiments, the active material comprises a natural extract and/or an essential oil.

In some embodiments, the active material is selected from the group consisting of fragrance, pro-fragrance, malodor counteractive agent, and combinations thereof. In some embodiments, the active material comprises a fragrance. In some embodiments, the active material comprises a low logP fragrance having a logP value ranging from 0.5 to 2.2, and the amount of the low logP fragrance is from 2% to 18% by weight based on the weight of the active material. In some embodiments, the amount of the low logP fragrance is at least 1.5%, 2%, 3%, 4%, 5%, 6%, 7%, or 8% by weight based on the weight of the active material. In some embodiments, the amount of the low logP fragrance is no more than 18%, 17%, 16%, 15%, 14%, 13%, 12%, 11%, 10%, 9%, 8%, or 7% by weight based on the weight of the active material. In some embodiments, the amount of the low logP fragrance ranges from 2% to 15%, 4% to 12%, or 5% to 10% by weight based on the weight of the active material.

In some embodiments, the low logP fragrance has a water solubility of at least 1 g/L, 1.5 g/L, 2 g/L, 2.5 g/L, 3 g/L, or 3.5 g/L, measured at 22.5 °C. In some embodiments, the low logP fragrance is selected from the group consisting of ethyl vanillin, coumarin, 4-(4-hydroxyphenyl)butan-2-one (CAS Number: 5471-51-2), p-anisaldehyde, 2-ethyl-3-hydroxy-4H-pyran-4-one (ethyl maltol, CAS Number: 4940-11-8), benzaldehyde, cinnamaldehyde, and combinations thereof. In some embodiments, the low logP fragrance comprises ethyl vanillin.

Low logP fragrances are difficult to be encapsulated due to their high water solubility. They also tend to leak out of the microcapsules, which leads to poor capsule stability and low performance of the capsules on dry stages during laundry. Moreover, ethyl vanillin can cause discoloration of the slurry and therefore, also to the commercial product in which the slurry is added. It has now been found that the core-shell microcapsules of the present disclosure are able to encapsulate active materials (e.g., fragrances) that comprise high levels of low logP fragrance ingredients. Accordingly, the present disclosure provides a core-shell microcapsule slurry comprising: (a) core-shell microcapsules having diameter of 1 to 100 microns; (b) a dispersant comprising denatured pea protein; and (c) a hydrocolloid comprising gum Arabic. In some embodiments, the hydrocolloid comprises gum Arabic added to an aqueous phase before an emulsification step during formation of the slurry. In some embodiments, the microcapsule slurry is an aqueous suspension of the microcapsule. In some embodiments, the microcapsule slurry is white. The microcapsule slurry may be used directly in a consumer product. The microcapsule slurry may also be washed, coated, dried (*e.g*., spray-dried) and/or combined with one or more other microcapsules, active materials, and/or carrier materials. A core-shell microcapsule comprises a core of the microcapsule (i.e., core or microcapsule core) and a shell of the microcapsule (i.e., shell or microcapsule shell). The core of the microcapsule comprises an active material, and the shell of the microcapsule comprises a self-condensed polyisocyanate. The microcapsules in the present disclosure don't have to be perfectly spherical. The term "diameter", as used herein with respect to a microcapsule, means the diameter of a sphere having the same volume as the microcapsule.

The core of the microcapsule comprises an active material. The active material comprises a low logP fragrance having a logP value ranging from 0.5 to 2.2, and the amount of the low logP fragrance is from 2% to 18% by weight based on the weight of the active material. In some embodiments, the core of the microcapsule further comprises an adjunct core material such as a solvent, an emollient, and/or a core modifier material. Examples of adjunct core materials include nanoscale solid particulate materials, polymeric core modifiers, solubility modifiers, density modifiers, stabilizers, humectants, viscosity modifiers, pH modifiers, or a combination thereof. Suitable examples of adjunct core materials also include those described in WO 2016/049456 and US 2016/0158121. In some embodiments, the solvent comprises caprylic/capric triglyceride. In some embodiments, the solvent comprises benzyl benzoate. In some embodiments, the adjunct core material can also be present in the wall or outside the capsule in the slurry. In some embodiments, the adjunct core material can be present in the core in an amount of from 0.01% to 25% (*e.g.,* from 0.5% to 10%) by weight of the capsule.

The shell of the microcapsule is formed by the self-polymerization of a polyisocyanate in the presence of water, a denatured pea protein and a gum Arabic. In some embodiments, the polyisocyanate comprises or is trimethylol propane-adduct of xylylene diisocyanate. It has now been found that a polyisocyanate, such as trimethylol propane-adduct of xylylene diisocyanate, when reacted with water to form an amine group, can self-polymerize in the presence of a denatured pea protein (as dispersant) and form a wall material suitable for encapsulation of active materials. In some embodiments, the polyisocyanate does not crosslink with the denatured pea protein. Rather, the denatured pea protein appears to function as a scaffold to facilitate the self-polymerization reaction of the polyisocyanate to form a wall polymer encapsulating the active material. Moreover, addition of gum Arabic prior to emulsification facilitates dissolution of denatured pea protein in the aqueous phase thereby preventing aggregation of the same. In some embodiments, the shell of the microcapsule comprises a single type of polymer (i.e., self-condensed polyisocyanate). In this regard, the shell of the microcapsule is formed by the self-polymerization of one or more polyisocyanates. In some embodiments, the self-condensed polyisocyanate is formed in the absence of an exogenous (i.e., added) crosslinking agent such as polyamine and polyalcohol. In some embodiments, the shell of the microcapsule is substantially free of or free of a biopolymer (e.g., pea protein and gum Arabic) and/or a polyelectrolyte. In some embodiments, the microcapsule shell comprises no more than 15%, 10%, 5%, 3%, 1%, 0.5%, 0.2%, or 0.1% biopolymer, relative to the total weight of the microcapsule shell. In some embodiments, the microcapsule shell comprises no more than 15%, 10%, 5%, 3%, 1%, 0.5%, 0.2%, or 0.1% polyelectrolyte, relative to the total weight of the microcapsule shell.

In some embodiments, the microcapsule shell is biodegradable. The term "biodegradable" as used herein with respect to a material, such as a microcapsule shell as a whole or a polymer of the microcapsule shell, means that the material has no real or perceived health and/or environmental issues, and is capable of undergoing and/or does undergo physical, chemical, thermal, microbial, biological and/or UV or photo-degradation. Ideally, a microcapsule shell and/or polymer is deemed "biodegradable" when the microcapsule shell and/or polymer passes one or more of the following tests including: a respirometry biodegradation method in aquatic media, available from Organization for Economic Cooperation and Development (OECD), International Organization for Standardization (ISO) and the American Society for Testing and Material (ASTM) tests including, but not limited to OECD 301F or 310 (Ready biodegradation), OECD 302 (inherent biodegradation), ISO 17556 (solid stimulation studies), ISO 14851 (fresh water stimulation studies), ISO 18830 (marine sediment stimulation studies), OECD 307 (soil stimulation studies), OECD 308 (sediment stimulation studies), and OECD 309 (water stimulation studies). Preferably, the microcapsules are readily biodegradable as determined using a respirometry biodegradation method in aquatic media, the OECD 301F or OECD 310 test. More preferably, the microcapsule shell and/or polymer is biodegradable if the shell and/or polymer has a biodegradation rate of at least 20%, 30%, 40%, 50%, 60%, 65%, 70%, 75%, 80%, 85%, 90%, 95% or 98%, based on the weight of the shell and/or polymer, within 60 days according to the OECD301F or OECD310 tests, or most preferably a biodegradability of at least 20% within 60 days according to OECD301F test.

In some embodiments, the shell of the microcapsule has a biodegradation rate of at least 20%, 30%, 40%, 50%, 60%, 65%, 70%, 75%, 80%, 85%, 90%, 95% or 98%, based on the weight of the shell, within 60 days according to OECD301F or OECD310. In some embodiments, the shell of the microcapsule has a biodegradation rate of at least 20%, based on the weight of the shell, within 60 days according to OECD301F or OECD310.

The shell of the microcapsules comprises a self-condensed polyisocyanate. In some embodiments, the amount of the self-condensed polyisocyanate ranges from 0.1% to 10%, preferably from 0.1% to 8%, more preferably from 0.2% to 5%, and even more preferably from 1.5% to 3.5% or from 0.1% to 5%, all by weight based on the weight of the core-shell microcapsule slurry. In some embodiments, the amount of the self-condensed polyisocyanate is no more than 1%, 0.9%, 0.8%, 0.7%, 0.6%, 0.5%, 0.4%, 0.3%, or 0.2%, by weight based on the weight of the core-shell microcapsule slurry.

In some embodiments, the core-shell microcapsules have diameter ranging from 0.1 micron to 1000 microns (e.g., from 0.5 micron to 500 microns, from 1 micron to 200 microns, from 1 micron to 100 microns, or from 1 micron to 50 micron). In some embodiments, the core-shell microcapsules have diameter of at least 0.1 micron, 0.5 micron, 1 micron, 2 microns, 5 microns or 20 microns. In some embodiments, the core-shell microcapsules have diameter of no more than 1000 microns, 500 microns, 200 microns, 100 microns, 75 microns, 50 microns, 30 microns, 20 microns, 10 microns or 5 microns.

The core-shell microcapsule slurry comprises a microcapsule formation aid. In some embodiments, the microcapsule formation aid is a dispersant which facilitates the formation of a stable emulsion containing nano- or micro-sized oil drops to be encapsulated. Microcapsule formation aids can also improve the performance of the microcapsule by stabilizing capsules and/or their deposition to the target areas or releasing to the environment. Performance is measured by the intensity of the fragrance release during various touchpoints of the user experience, such as the pre-rub and post-rub phases in a laundry experience. The pre-rub phase is the phase when the microcapsules have been deposited on the cloth, *e.g.,* after a fabric softener containing microcapsules has been used during the wash cycle. The post-rub phase is after the microcapsules have been deposited on the cloth and the microcapsules are broken by friction or other similar mechanisms. The amount of the microcapsule formation aid can be from 0.1% to 40%, from 0.1% to 10%, or from 0.1% to 5%, by weight based on the weight of the microcapsule.

Examples of microcapsule formation aids include polyvinyl pyrrolidone, polyvinyl alcohol, poly(styrene sulfonate), carboxymethyl cellulose, sodium salt of naphthalene sulfonate condensate, co-polymer of ethylene and maleic anhydride, alginate, hyaluronic acid, poly(acrylic acid), carboxymethylcellulose, copolymers of acrylic acid and acrylamide, copolymer of acrylamide and acrylamidopropyltrimonium chloride, terpolymers of (acrylic acid, acrylamide, and acrylamidopropyltrimonium chloride), partially or completely hydrolyzed polyvinyl acetate polymers (*i.e*., polyvinyl alcohol), or a combination thereof.

Other microcapsule formation aids include water-soluble salts of alkyl sulfates, alkyl ether sulfates, alkyl isothionates, alkyl carboxylates, alkyl sulfosuccinates, alkyl succinamates, alkyl sulfate salts such as sodium dodecyl sulfate, alkyl sarcosinates, alkyl derivatives of protein hydrolysates, acyl aspartates, alkyl or alkyl ether or alkylaryl ether phosphate esters, sodium dodecyl sulphate, phospholipids or lecithin, or soaps, sodium, potassium or ammonium stearate, oleate or palmitate, alkylarylsulfonic acid salts such as sodium dodecylbenzenesulfonate, sodium dialkylsulfosuccinates, dioctyl sulfosuccinate, sodium dilaurylsulfosuccinate, poly(styrene sulfonate) sodium salt, isobutylene-maleic anhydride copolymer, sodium alginate, cellulose sulfate and pectin, isobutylene-maleic anhydride copolymer, gum Arabic, carrageenan, pectic acid, tragacanth gum, almond gum and agar, semi-synthetic polymers such as sulfated cellulose, sulfated methylcellulose, carboxymethyl starch, phosphated starch, lignin sulfonic acid, synthetic polymers such as maleic anhydride copolymers (including hydrolysates thereof), polyacrylic acid, polymethacrylic acid, acrylic acid butyl acrylate copolymer or crotonic acid homopolymers and copolymers, vinylbenzenesulfonic acid or 2-acrylamido-2-methylpropanesulfonic acid homopolymers and copolymers, and partial amide or partial ester of such polymers and copolymers, carboxymodified polyvinyl alcohol, sulfonic acid-modified polyvinyl alcohol and phosphoric acid-modified polyvinyl alcohol, phosphated or sulfated tristyrylphenol ethoxylates.

In some embodiments, the microcapsule formation aid is a surfactant. Examples of surfactants include, but are not limited to, sulfonated naphthalene-formaldehyde condensates sold under the tradename MORWET^{®} D425 (sodium salt of alkylnaphthalenesulfonate formaldehyde condensate, Akzo Nobel, Fort Worth, TX); partially hydrolyzed polyvinyl alcohols sold under the tradenames MOWIOL^{®}, *e.g*., MOWIOL^{®} 3-83 (Air Products), or SELVOL^{®} 203 (Sekisui), or polyvinyl alcohols such as Ultalux FP, Ultalux FA, Ultalux AD, OKS-8089 (Sourus); ethylene oxide-propylene oxide block copolymers or poloxamers sold under the tradenames PLURONIC^{®}, SYNPERONIC^{®} or PLURACARE^{®} materials (BASF); sulfonated polystyrenes sold under the tradename FLEXAN^{®} II (Akzo Nobel); ethylene-maleic anhydride polymers sold under the tradename ZEMAC^{®} (Vertellus Specialties Inc.); copolymer of acrylamide and acrylamidopropyltrimonium chloride sold under the tradename SALCARE^{®} SC 60 (BASF); and polyquaternium series such as Polyquaternium 11 ("PQ11;" a copolymer of vinyl pyrrolidone and quaternized dimethylaminoethyl methacrylate; sold by BASF as Luviquat PQ1 1 AT 1). Surfactant MOWIOL^{®} 3-83 has a viscosity of 2-4 mPa·S *(e.g.,* 3 mPa S), a degree of hydrolysis of 80-85% (*e.g.,* 83%), an ester value of 170-210 mg KOH/g *(e.g.,* 190 mg KOH/g), and a residual unhydrolyzed acetyl content of 13-18% *(e.g.,* 15%). In certain aspects, the surfactant is a sulfonated polystyrene, *e.g*., the high molecular weight polystyrene sulfonate, sodium salt sold under the tradename FLEXAN^{®} II.

In some embodiments, the microcapsule formation aid is a processing aid such as a hydrocolloid, which can improve the colloidal stability of the slurry against coagulation, sedimentation and creaming. In some embodiments, the hydrocolloid is added to the aqueous phase before an emulsification step during formation of the slurry. The term "hydrocolloid" refers to a broad class of water-soluble or water-dispersible polymers having anionic, cationic, zwitterionic or non-ionic character. Suitable hydrocolloids include, but are not limited to, polycarbohydrates, such as starch, modified starch, dextrin, maltodextrin, and cellulose derivatives, and their quaternized forms; natural gums such as alginate esters, carrageenan, xanthan, agar-agar, pectins, pectic acid, gum Arabic, gum tragacanth and gum karaya, guar gums and quaternized guar gums; gelatin, protein hydrolysates and their quaternized forms, synthetic polymers and copolymers, such as poly(vinyl pyrrolidone-co-vinyl acetate), poly(vinyl alcohol-co-vinyl acetate), poly((met)acrylic acid), poly(maleic acid), poly(alkyl(meth)acrylate-co-(meth)acrylic acid), poly(acrylic acid-co-maleic acid)copolymer, poly(alkyleneoxide), poly(vinylmethylether), poly(vinylether-co-maleic anhydride), and the like, as well as poly-(ethyleneimine), poly((meth)acrylamide), poly(alkyleneoxide-co-dimethylsiloxane), poly(amino dimethylsiloxane), Ultrez 20 (Acrylates/C10-30 Alkyl Acrylate Crosspolymer), cross-linked homopolymer of acrylic acid polymerized in a cyclohexane and ethyl acetate co-solvent system sold under the tradename CARBOPOL^{®} Ultrez 30, acrylates copolymer sold under the tradename ACULYN^{®} Excel (Acrylates Copolymer), crosslinked polyacrylic acid polymer sold under the tradename CARBOPOL^{®} 981 (Carbomer), and the like, and their quaternized forms. In certain aspects, the core-shell microcapsule slurry is prepared in the presence of gum Arabic as a hydrocolloid. In certain aspects, the core-shell microcapsule slurry comprises a hydrocolloid comprising gum Arabic.

In some embodiments, the microcapsule formation aid can be used in combination with carboxymethyl cellulose ("CMC"), polyvinylpyrrolidone, polyvinyl alcohol, alkylnaphthalenesulfonate formaldehyde condensates, and/or a surfactant during processing to facilitate capsule formation. Examples of surfactants that can be used in combination with the microcapsule formation aid include, but are not limited to, cetyl trimethyl ammonium chloride (CTAC), poloxamers sold under the tradenames PLURONIC^{®} (*e.g*., PLURONIC^{®} F127), PLURAFAC^{®} (*e.g*., PLURAFAC^{®} F127), or Miranet-N, saponins sold under the tradename Q-NATURALE^{®} (National Starch Food Innovation); or a gum Arabic such as Seyal or Senegal.

In certain aspects, the CMC polymer has a molecular weight ranging between about 90,000 Daltons to 1,500,000 Daltons, preferably between about 250,000 Daltons to 750,000 Daltons and more preferably between 400,000 Daltons to 750,000 Daltons. The CMC polymer has a degree of substitution between about 0.1 to about 3, preferably between about 0.65 to about 1.4, and more preferably between about 0.8 to about 1.0. The CMC polymer can be present in the microcapsule slurry in an amount of from about 0.1% to about 2% and preferably from about 0.3% to about 0.7%, based on the weight of the microcapsule slurry. In other aspects, polyvinylpyrrolidone used in this disclosure is a water-soluble polymer and has a molecular weight of from 1,000 to 10,000,000 Daltons. Suitable polyvinylpyrrolidones include polyvinylpyrrolidone K12, K15, K17, K25, K30, K60, K90, or a combination thereof. The amount of polyvinylpyrrolidone can be 2-50%, 5-30%, or 10-25% by weight of the microcapsule slurry. Commercially available alkylnaphthalenesulfonate formaldehyde condensates include MORWET^{®} D-425, which is a sodium salt of naphthalene sulfonate condensate by Akzo Nobel, Fort Worth, TX.

In some embodiments, the microcapsule formation aid is a food-grade dispersant. The term "food-grade dispersant" refers to a dispersant having a quality as fit for human consumption in food. They can be natural or non-natural dispersants. A natural dispersant refers to a dispersant that is naturally occurring and comes from a nature source. Natural dispersants include their derivatives which can be salted, desalted, deoiled, fractionated, or modified using a natural enzyme or microorganism. A non-natural dispersant is a chemically synthesized dispersant by a chemical process that does not involve an enzymatic modification.

Natural dispersants include quillaja saponin, lecithins, gum Arabic, pectin, carrageenan, chitosan, chondroitin sulfate, modified cellulose, cellulose gum, modified starch, whey protein, pea protein, egg white protein, silk protein, gelatin of fish, proteins of porcine or bovine origin, ester gum, fatty acids, or a combination thereof. In certain aspects, the microcapsule slurry is prepared in the presence of denatured protein, e.g., a denatured pea protein, as a dispersant. In certain aspects, the core-shell microcapsule slurry comprises a dispersant comprising a denatured protein such as a denatured pea protein, in particular a denatured pea protein isolate.

In some embodiments, the natural dispersant is a plant storage protein. Plant storage proteins are proteins that accumulate in various plant tissues and function as biological reserves of metal ions and amino acids. Plant storage proteins can be classified into two classes: seed or grain storage proteins and vegetative storage proteins. Seed/grain storage proteins are a set of proteins that accumulate to high levels in seeds/grains during the late stages of seed/grain development, whereas vegetative storage proteins are proteins that accumulate in vegetative tissues such as leaves, stems and, depending on plant species, tubers. During germination, seed/grain storage proteins are degraded and the resulting amino acids are used by the developing seedlings as a nutritional source. In some aspects, the dispersant used in the preparation of a microcapsule is a leguminous storage protein, in particular a protein extracted from soy, lupine, pea, chickpea, alfalfa, horse bean, lentil, haricot bean, or a combination thereof. In some embodiments, the plant storage protein is a pea protein.

Pea protein includes pea protein isolate, pea protein concentrate, or a combination thereof. Pea protein isolates and concentrates are generally understood to be composed of several proteins. For example, pea protein isolates and concentrates can include legumin, vicilin and convicilin proteins. The term "pea protein" is also intended to include a partially or completely modified or denatured pea protein. Individual plant storage protein (*e.g*., legumin, vicilin, or convicilin) can also be used in the preparation of microcapsules of this disclosure.

Ideally, the pea protein of the present disclosure is denatured, preferably without causing gelation of the pea protein. Exemplary conditions for protein (e.g., pea protein) denaturation include, but are not limited to, exposure to heat or cold, changes in pH, exposure to denaturing agents such as detergents, urea, or other chaotropic agents, or mechanical stress including shear. In some aspects, the pea protein is partially denatured, *e.g.,* 50%, 60%, 70%, 80% or 85% denatured, based on the total weight of pea protein. In other aspects, the pea protein is substantially or completely denatured, *e.g.,* at least 90%, 91%, 92%, 93%, 94%, 95%, 96%, 97%, 98%, 99% or 100% denatured, based on the total weight of pea protein. For example, when an 8% pea protein solution (w/v) is used, the solution can be treated at a temperature of 80 °C to 90 °C for 20 to 30 minutes (or preferably 85 °C for 25 minutes) to yield a substantially denatured pea protein. Accordingly, depending on the degree of denaturation desired, it will be appreciated that higher temperatures and shorter times can also be employed.

In particular, it has been found that chaotropic agents are particularly useful in providing a denatured protein (e.g., denatured pea protein) of use in the preparation of the microcapsules of the present disclosure. As is conventional in the art, a chaotropic agent is a compound which disrupts hydrogen bonding in aqueous solution, leading to increased entropy. Generally, this reduces hydrophobic effects which are essential for three dimensional structures of proteins. Chaotropes can be defined by having a positive chaotropic value, *i.e.,* kJ kg⁻¹ mole on the Hallsworth Scale. Examples of chaotropicity values are, for example, CaCh +92.2 kJ kg⁻¹, MgCh kJ kg⁻¹ +54.0, butanol +37.4 kJ kg⁻¹, guanidine hydrochloride +31.9 kJ kg⁻¹, and urea +16.6 kJ kg⁻¹. In certain aspects, the chaotropic agent is a guanidinium salt, *e.g*., guanidinium sulphate, guanidinium carbonate, guanidinium nitrate or guanidinium chloride. In particular aspects, the pea protein is partially or completely denatured with guanidine carbonate.

In addition to natural dispersants, non-natural dispersants are of use in the preparation of the microcapsules of the present disclosure. Non-natural dispersants include N-lauroyl-L-arginine ethyl ester, sorbitan esters, polyethoxylated sorbitan esters, polyglyceryl esters, fatty acid esters, or a combination thereof.

Other food safe dispersants can also be used in the microcapsule slurry of the present disclosure. Examples include ammonium phosphatides, acetic acid esters of mono- and diglycerides (Acetem), lactic acid esters of mono- and diglycerides of fatty acids (Lactem), citric acid esters of mono and diglycerides of fatty acids (Citrem), mono and diacetyl tartaric acid esters of mono and diglycerides of fatty acids (Datem), succinic acid esters of monoglycerides of fatty acids (SMG), ethoxylated monoglycerides, sucrose esters of fatty acids, sucroglycerides, polyglycerol polyricinoleate, propane-1,2-diol esters of fatty acids, thermally oxidized soybean oil interacted with mono- or diglycerides of fatty acids, sodium stearoyl lactylate (SSL), calcium stearoyl lactylate (CSL), stearyl tartrate, polyglycerol esters of interesterified castor oil acid (E476), sodium stearoyllatylate, sodium lauryl sulfate, polyoxyethylated hydrogenated castor oil (for instance, such sold under the tradename CREMO-PHOR^{®}), block copolymers of ethylene oxide and propylene oxide (for instance as sold under the tradename PLURONIC^{®}, polyoxyethylene fatty alcohol ethers, and polyoxyethylene stearic acid ester.

In some embodiments, the core-shell microcapsule slurry also comprises a rheology modifier (*e.g*., xanthan gum), a preservative, an emulsifier, or a combination thereof. In some embodiments, the core-shell microcapsule slurry also comprises a rheology modifier. One or more rheology modifiers or viscosity control agents can be added to the microcapsule slurry to achieve a desired viscosity of the slurry so that the microcapsule is dispersed in the slurry for a pro-longed period of time. During capsule preparation, the rheology modifier is preferably added prior to the emulsification of the aqueous phase and oil phase and is typically dispersed homogeneously in the microcapsule slurry and outside of the microcapsule wall of the microcapsules. Suitable rheology modifiers include an acrylate copolymer, a cationic acrylamide copolymer, a polysaccharide, or a combination thereof. Preferably, the addition of a rheology modifier to the slurry provides a slurry having a viscosity of less than 600 cps or less than 580 cps as measured at shear rate of 21 s⁻¹.

Commercially available acrylate copolymers include those under the tradename ACULYN^{®} (from Dow Chemical Company) such as ACULYN^{®} 22 (a copolymer of acrylates and stearth-20 methacrylate), ACULYN^{®} 28 (a copolymer of acrylate and beheneth-25 methacrylate), ACULYN^{®} 33 (a copolymer of acrylic acid and acrylate), ACULYN^{®} 38 (a cross polymer of acrylate and vinyl neodecanoate), and ACULYN^{®} 88 (a cross polymer of acrylate and steareth-20 methacrylate). Particularly useful acrylate copolymers are anionic acrylate copolymer such as ACULYN^{®} 33, an alkali-soluble anionic acrylic polymer emulsion (ASE), which is synthesized from acrylic acid and acrylate comonomers through emulsion polymerization. Acrylate copolymers sold under the tradename CARBOPOL^{®} are also suitable for use in this invention. Examples are CARBOPOL^{®} ETD 2020 polymer (a cross polymer of acrylate and C₁₀-C₃₀ alkyl acrylate), CARBOPOL^{®} ETD 2691, and CARBOPOL^{®} ETD 2623 (a crosslinked acrylate copolymer).

Polysaccharides are another class of agents suitable as rheology modifiers. In certain aspects, polysaccharides that are useful as rheology modifiers include starches, pectin, and vegetable gums such as alginin, guar gum, locust bean gum, and xanthan gum, *e.g*., xanthan gum sold under the tradename KELTROL^{®} T (80-mesh food-grade), commercially available from CP Kelco, Atlanta, GA. Preferably, the rheology modifier comprises or is a xanthan gum.

In some embodiments, the active material is a fragrance and the microcapsule slurry has (a) less than 0.3% or less than 0.25% of a non-encapsulated fragrance, based on total weight of the fragrance in the slurry, (b) a viscosity of less than 600 cps or less than 580 cps as measured at shear rate of 21 s⁻¹, or (c) a combination of (a) and (b).

In some embodiments, the core-shell microcapsule slurry also comprises a capsule deposition aid. The amount of the capsule deposition aid in the slurry can range from 0.01% to 25%, more preferably from 5% to 20%, by weight based on the weight of the microcapsule. The capsule deposition aid can be added during the preparation of the capsules or it can be added after the capsules have been made.

Capsule deposition aids are used to aid in deposition of capsules to surfaces such as fabric, hair or skin. Examples of capsule deposition aids include anionically, cationically, nonionically, or amphoteric water-soluble polymers. Suitable capsule deposition aids include polyquaternium-4, polyquaternium-5, polyquaternium-6, polyquaternium-7, polyquaternium-10, polyquaternium-16, polyquaternium-22, polyquaternium-24, polyquaternium-28, polyquaternium-39, polyquaternium-44, polyquaternium-46, polyquaternium-47, polyquaternium-53, polyquaternium-55, polyquaternium-67, polyquaternium-68, polyquaternium-69, polyquaternium-73, polyquaternium-74, polyquaternium-77, polyquaternium-78, polyquaternium-79, polyquaternium-80, polyquaternium-81, polyquaternium-82, polyquaternium-86, polyquaternium-88, polyquaternium-101, polyvinylamine, polyethyleneimine, polyvinylamine and vinylformamide copolymer, an acrylamidopropyltrimonium chloride/acrylamide copolymer, a methacrylamidopropyltrimonium chloride/acrylamide copolymer, polymer comprising units derived from polyethylene glycol and terephthalate, polyester, polymer derivable from dicarboxylic acids and polyols, or a combination thereof. Other suitable capsule deposition aids include those described in WO 2016/049456, pages 13-27. Additional capsule deposition aids are described in US 2013/0330292, US 2013/0337023, and US 2014/0017278.

In some embodiments, the core-shell microcapsule slurry also comprises a preservative. One or more preservatives can be added to the microcapsule slurry to prevent damage or inadvertent growth of microorganisms for a specific period of time thereby increasing shelf life. The preservative can be any organic preservative that does not cause damage to the microcapsule slurry. Suitable water-soluble preservatives include organic sulfur compounds, halogenated compounds, cyclic organic nitrogen compounds, low molecular weight aldehydes, parabens, propanediol materials, isothiazolinone, quaternary compounds, and benzoates. Examples of preservatives include low molecular weight alcohols, dehydroacetic acids, phenyl and phenoxy compounds, or a combination thereof.

A non-limiting example of commercially available water-soluble preservative is a mixture of about 77% 5-chloro-2-methyl-4-isothiazolin-3-one and 23% 2-methyl-4-isothiazolin-3-one. Additional antibacterial preservatives include a 1.5% aqueous solution under the tradename KATHON^{®} CG of Rohm &Haas; 5-bromo available under the tradename BRONIDOX L^{®} of Henkel; 2-bromo-2-nitro-1,3-propanediol available under the tradename BRONOPOL^{®} of Inorex; 1,1'-Hexamethylenebis (5-(p-chlorophenyl) biguanide) and salts thereof, such as acetates and digluconates; 1,3-bis (hydroxy) available under the tradename GLYDANT PLUS^{®} from Ronza; glutaraldehyde; ICI Polyaminopropylbiguanide; dehydroacetic acid; and 1,2-Benzisothiazolin-3-one sold under the tradename PROXEL^{®} GXL.

The microcapsule slurry of this disclosure is shown to be an effective delivery system capable of delivering a fragrance in a consumer product such as a fabric conditioner. Additionally, the microcapsule slurry also finds utility in a wide range of consumer applications, e.g., personal care products including shampoos, hair conditioners, hair rinses, hair refreshers; personal wash such as bar soaps, body wash, personal cleaners and sanitizers; fabric care such as fabric refreshers, softeners and dryer sheets, ironing water, industrial cleaners, liquid and powder detergent including unit dose capsules, rinse conditioners, and scent booster products; fine fragrances such as body mist and Eau De Toilette products; deodorants; roll-on products, and aerosol products.

The present disclosure also provides a method for producing a core-shell microcapsule slurry as described in this disclosure. The method comprises: (a) preparing an aqueous phase by (i) denaturing a pea protein, (ii) adjusting the pH of the aqueous phase to below 6, and (iii) adding gum Arabic as a hydrocolloid to the aqueous phase; (b) preparing an oil phase comprising an active material and a polyisocyanate; (c) emulsifying the oil phase into the aqueous phase to form a slurry comprising core-shell microcapsules; and (d) curing the shell of the microcapsules at a temperature below 80 °C; wherein the active material comprises a low logP fragrance having a logP value ranging from 0.5 to 2.2, the amount of the low logP fragrance is from 2% to 18% by weight based on the weight of the active material, and the core-shell microcapsule slurry is white.

As demonstrated herein, a polyisocyanate, when reacted with water to form an amine, will self-polymerize in the presence of a denatured pea protein as dispersant and form a wall material suitable for encapsulation of active materials in a core-shell microcapsule. Not wishing to be bound by theory, it is posited that the denatured pea protein provides a scaffold that facilitates self-polymerization of the polyisocyanate. Advantageously, the inclusion of denatured pea protein provides for the use of reduced levels of polyisocyanate and improves the sustainability and biodegradability of the resulting core-shell microcapsules. Moreover, desirable microcapsule properties such as good dry performance, low discoloration and/or reduced aggregation or agglomeration can be achieved by adjusting the pH of the emulsion or slurry to below 6 and/or curing the microcapsule shell at a temperature below 80 °C.

A pea protein can be denatured in an aqueous phase by being exposed to heat or cold, changes in pH, denaturing agents such as detergents, urea, or other chaotropic agents, or mechanical stress including shear. In some embodiments, the pea protein is denatured by heating an aqueous pea protein solution to a temperature of from 80 °C to 170 °C, or from 80 °C to 140 °C, or from 80 °C to 120 °C, or from 80 °C to 105 °C, or from 80 °C to 90 °C. In some embodiments, the pea protein is denatured by a denaturing agent. In some embodiments, the pea protein is denatured by a chaotropic agent. In some embodiments, the chaotropic agent comprises or is a guanidinium salt. In some embodiments, the guanidinium salt is selected from the group consisting of guanidinium sulphate, guanidinium carbonate, guanidinium nitrate, guanidinium chloride, and mixtures thereof. In some embodiments, the chaotropic agent comprises guanidine carbonate. In some embodiments, the denaturing step (a)(i) comprises mixing a pea protein and guanidine carbonate in an aqueous solution (e.g., aqueous phase).

The pH of the aqueous phase (e.g., the aqueous solution containing the mixture of pea protein and guanidine carbonate) is adjusted to below 6 or no more than 6. In some embodiments, the pH of the aqueous phase is adjusted to below 5.5 or no more than 5.5. In some embodiments, the pH of the aqueous phase is adjusted to a range of from 2 to 6, or from 3 to 5.5, or from 3.5 to 4.5, or from 3.8 to 4.2. Gum Arabic is added to the aqueous phase as a hydrocolloid.

The oil phase can be prepared by mixing an active material and a polyisocyanate. In some embodiments, an adjunct core material (e.g., a solvent) is also mixed with the active material and the polyisocyanate. In some embodiments, the polyisocyanate is dissolved in a solution comprising a solvent (e.g., caprylic/capric triglyceride) and an active material. In some embodiments, the active material is selected from the group consisting of fragrance, pro-fragrance, malodor counteractive agent, and combinations thereof. The active material comprises a low logP fragrance having a logP value ranging from 0.5 to 2.2, and the amount of the low logP fragrance is from 2% to 18% by weight based on the weight of the active material. In some embodiments, the active material is a fragrance and the slurry has (a) less than 0.3% or less than 0.25% of a non-encapsulated fragrance, based on total weight of the fragrance, (b) a viscosity of less than 600 cps or less than 580 cps as measured at shear rate of 21 s⁻¹, or (c) a combination of (a) and (b). In some embodiments, the amount of the polyisocyanate used in the preparation of microcapsule and microcapsule slurry is from 0.1% to 8% based on the weight of the core-shell microcapsule slurry. In some embodiments, the amount of the polyisocyanate used in the preparation of microcapsule and microcapsule slurry ranges from 0.1% to 10%, or from 0.1% to 8%, or from 0.2% to 5%, or from 1.5% to 3.5%, or from 0.1% to 5%, by weight based on the weight of the core-shell microcapsule slurry. In some embodiments, the amount of the polyisocyanate used in the preparation of microcapsule and microcapsule slurry is no more than 1%, 0.9%, 0.8%, 0.7%, 0.6%, 0.5%, 0.4%, 0.3%, or 0.2%, by weight based on the weight of the core-shell microcapsule slurry. In some embodiments, the polyisocyanate comprises or is trimethylol propane-adduct of xylylene diisocyanate.

In step (c), the oil phase is emulsified with the aqueous phase to form a slurry comprising core-shell microcapsules. In some embodiments, the slurry is an emulsion. In step (d), the shell of the microcapsules is cured at a temperature below 80 °C (*e.g.,* in a range of from 63 °C to 67 °C). The term "curing", as used herein, means a toughening or hardening process of a polymer brought about by heat, chemical additives, and/or light radiation. In some embodiments, the microcapsule shell is cured at an elevated temperature. In some embodiments, the microcapsule shell is cured at a temperature below 80 °C, or below 70 °C. In some embodiments, the microcapsule shell is cured at a temperature ranging from 15 °C to 80 °C, or from 55 °C to 65 °C, or from 55 °C to 70 °C, or from 55 °C to 80 °C, or from 63 °C to 67 °C. In some embodiments, the microcapsule shell can be cured for 1 minute to 10 hours, or 0.1 hours to 5 hours, or 0.2 hours to 4 hours, or 0.5 hours to 3 hours. In some embodiments, the microcapsule slurry can be heated to the desired curing temperature at a linear rate of 0.5 to 20 °C per minute (e.g., 1 to 5 °C per minute, 2 to 8 °C per minute, or 2 to 10 °C per minute).

In some embodiments, the method for producing the microcapsule slurry further comprises adding a rheology modifier, a preservative, an emulsifier, or a combination thereof into the microcapsule slurry. In some embodiments, the rheology modifier (*e.g*., xanthan gum) is added into the aqueous phase prior to step (c).

In some embodiments, no catalyst, such as 1,4-diazabicylo[2,2,2]octane (DABCO), N-methylimidazole, diaminobicycloctane, 2,2'-dimorpholinodiethylether, is used for the formation of the shell of the microcapsules. In some embodiments, the amount of the catalyst for the shell formation is no more than 50 wppm (parts per million by weight), 20 wppm, 10 wppm, 5 wppm, 2 wppm, 1 wppm, 0.5 wppm, or 0.2 wppm, based on the weight of the microcapsule slurry.

The microcapsule slurry of this disclosure can be formulated into a microcapsule delivery system for use in consumer products. The microcapsule delivery system can be a microcapsule slurry suspended in an external solvent (*e.g*., water, ethanol, or a combination thereof), wherein the microcapsule is present at a level of 0.1% to 80% (e.g., 70-75%, 40-55%, 50-90%, 1% to 65%, or 5% to 45%) by weight of the microcapsule delivery system.

Alternatively, or in addition to, the microcapsule and its slurry prepared in accordance with the present disclosure can be subsequently purified. See US 2014/0017287. Purification can be achieved by washing the microcapsule slurry with water until a neutral pH is obtained.

The microcapsule delivery system can optionally contain one or more other delivery system such as polymer-assisted delivery compositions (see US 8,187,580), fiber-assisted delivery compositions (US 2010/0305021), cyclodextrin host guest complexes (US 6,287,603 and US 2002/0019369), pro-fragrances (WO 2000/072816 and EP 0922084), or a combination thereof. The microcapsule delivery system can also contain one or more (*e.g*., two, three, four, five or six or more) different microcapsules including different microcapsules of the present disclosure and other microcapsules such as aminoplasts, hydrogel, sol-gel, polyurea/polyurethane microcapsules, and melamine formaldehyde microcapsules. More exemplary delivery systems that can be incorporated are coacervate microcapsules (see WO 2004/022221) and cyclodextrin delivery systems (see WO 2013/109798 and US 2011/03085560).

The microcapsule slurry of this disclosure is well-suited for inclusion in any of a variety of consumer products where controlled release of active material (*e.g*., fragrances or flavors) is desired. The present disclosure also provides a consumer product comprising the core-shell microcapsule slurry of this disclosure. In some embodiments, the consumer product is selected from the group consisting of fabric conditioner, fabric softener, fabric refresher, liquid laundry detergent, powder detergent, scent booster, body wash, body soap, shampoo, hair conditioner, body spray, hair refresher spray, hair dye, hair moisturizer, skin moisturizer, hair treatment, antiperspirant, deodorant, skin treatment, insect repellant, candle, surface cleaner, bathroom cleaner, bleach, cat litter, refresher spray, pesticide, insecticide, herbicide, fungicide, paint, and combinations thereof.

### Applications

The microcapsule slurry and delivery systems of the present disclosure are well-suited for use, without limitation, in a laundry detergent, a liquid laundry detergent, a powder laundry detergent, a tablet laundry detergent, a laundry detergent bar, a laundry detergent cream, a hand wash laundry detergent, a fabric conditioner or softener, a fabric refresher, a scent booster, a shampoo, a hair conditioner, a bar soap, a shower gel, a body wash, an antiperspirant, a body spray, a body mist, a lotion, a candle or a textile.

More specifically, the microcapsules of the present disclosure can be used in the following products:
A) Fabric Care Products such as Rinse Conditioners (containing 1 to 30 weight % of a fabric conditioning active), Fabric Liquid Conditioners (containing 1 to 30 weight % of a fabric conditioning active), Tumble Drier Sheets, Fabric Refreshers, Fabric Refresher Sprays, Ironing Liquids, and Fabric Softener Systems such as those described in US 6,335,315, US 5,674,832, US 5,759,990, US 5,877,145, US 5,574,179, US 5,562,849, US 5,545,350, US 5,545,340, US 5,411,671, US 5,403,499, US 5,288,417, US 4,767,547 and US 4,424,134.

Liquid fabric softeners/fresheners contain at least one fabric softening agent present, preferably at a concentration of 1 to 30% *(e.g.,* 4% to 20%, 4% to 10%, and 8% to 15%) by weight of the liquid fabric softener/freshener. The ratio between the active material and the fabric softening agent can be 1:500 to 1:2 *(e.g.,* 1:250 to 1:4 and 1:100 to 1:8). As an illustration, when the fabric softening agent is 5% by weight of the fabric softener, the active material is 0.01% to 2.5%, preferably 0.02% to 1.25% and more preferably 0.1% to 0.63%. As another example, when the fabric softening agent is 20% by weight of the fabric softener, the active material is 0.04% to 10%, preferably 0.08% to 5% and more preferably 0.4% to 2.5%. The active material is a fragrance, malodor counteractant or a combination thereof. The liquid fabric softener can have 0.15% to 15% of capsules *(e.g.,* 0.5% to 10%, 0.7% to 5%, and 1% to 3%). When including capsules at these levels, the neat oil equivalent (NOE) in the softener is 0.05% to 5% *(e.g.,* 0.15% to 3.2%, 0.25% to 2%, and 0.3% to 1%).

Suitable fabric softening agents include cationic surfactants. Non-limiting examples are quaternary ammonium compounds (QAC) such as alkylated quaternary ammonium compounds, ring or cyclic quaternary ammonium compounds, aromatic quaternary ammonium compounds, diquaternary ammonium compounds, alkoxylated quaternary ammonium compounds, amidoamine quaternary ammonium compounds, ester quaternary ammonium compounds, or a combination thereof.

Fabric softening product includes an aqueous QAC which are characterized by:
a) The viscosity of the final product ranges from 5 to 300 cps @ 106s-1, preferable 20 to 150 cps;
b) The level of QAC ranges 0.5 to 20 wt%, preferably from 1 to 16 wt%, more preferably 6 to 12 wt% softening active. The preferred, typical cationic fabric softening components include water-insoluble quaternary-ammonium fabric softeners, the most commonly used having been di-long alkyl chain ammonium chloride or methyl sulfate. Preferred cationic softeners include but not limited to the following:
   a. rapidly biodegradable quaternary ammonium compounds which contain 1 or more ester bonds situated between the quaternary-ammonium group and the long alkyl chain (*e.g*., TEA ester quats, DEEDMAC and HEQ);
   b. Non-Ester quaternary ammonium compounds (*e.g*., ditallow dimethylammonium chloride (DTDMAC); dihydrogenated tallow dimethylammonium chloride; dihydrogenated tallow dimethylammonium methylsulfate; distearyl dimethylammonium chloride; dioleyl dimethylammonium chloride; dipalmityl hydroxyethyl methylammonium chloride; stearyl benzyl dimethylammonium chloride; tallow trimethylammonium chloride; hydrogenated tallow trimethylammonium chloride; C12-14 alkyl hydroxyethyl dimethylammonium chloride; C12-18 alkyl dihydroxyethyl methylammonium chloride; di(stearoyloxyethyl) dimethylammonium chloride (DSOEDMAC); di(tallowoyloxyethyl) dimethylammonium chloride; ditallow imidazolinium methylsulfate; 1-(2-tallowylamidoethyl)-2-tallowyl imidazolinium methyl sulfate.

A first group of quaternary ammonium compounds (QACs) suitable for use according to the present disclosure is represented by formula (I): 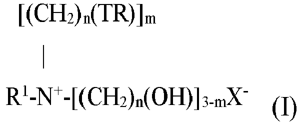 wherein each R is independently selected from a C₁-C₃₅ alkyl or alkenyl group; R¹ represents a C₁-C₄ alkyl, C₂-C₄ alkenyl or a C₁-C₄ hydroxyalkyl group; T is generally O-CO *(i.e.,* an ester group bound to R via its carbon atom), but may alternatively be CO-O *(i.e.,* an ester group bound to R via its oxygen atom); n is a number selected from 1 to 4; m is a number selected from 1, 2, or 3; and X is an anionic counter-ion, such as a halide or alkyl sulphate, *e.g*., chloride or methylsulphate. Di-esters variants of formula (I) (*i.e.,* m = 2) are preferred and typically have mono- and tri-ester analogues associated with them.

Especially preferred agents are preparations which are rich in the di-esters of triethanolammonium methyl sulfate, otherwise referred to as "TEA ester quats". Commercial examples include STEPANTEX^{®} UL85, ex Stepan, Prapagen^{™} TQL, ex Clariant, and Tetranyl^{™} AHT-1, ex Kao, (both di-[hardened tallow ester] of triethanolammonium methylsulphate), AT-1 (di-[tallow ester] of triethanolammonium methylsulphate), and L5/90 (di-[palm ester] of triethanolammonium methyl sulphate), both ex Kao, and REWOQUAT^{®} WE15 (a di-ester of triethanolammonium methyl sulphate having fatty acyl residues deriving from C₁₀-C₂₀ and C₁₆-C₁₈ unsaturated fatty acids), ex Evonik.

Also suitable are soft quaternary ammonium actives such as STEPANTEX^{®} VK90, STEPANTEX^{®} VT90, SP88 (ex-Stepan), Prapagen^{™} TQ (ex-Clariant), DEHYQUART^{®} AU-57 (ex-Cognis), REWOQUAT^{®} WE18 (ex-Degussa) and Tetranyl^{™} L190 P, Tetranyl^{™} L190 SP and Tetranyl^{™} L190 S (all ex-Kao).

A second group of QACs suitable for use according to the present disclosure is represented by formula (II):

(R¹)₂-N⁺-[(CH₂)ₙ-T-R²]₂X⁻ (II)

wherein each R¹ group is independently selected from C₁-C₄ alkyl, or C₂-C₄ alkenyl groups; and wherein each R² group is independently selected from C₈-C₂₈ alkyl or alkenyl groups; and n, T, and X- are as defined above. Preferred materials of this second group include bis(2tallwoyloxyethyl)dimethyl ammonium chloride and hardened versions thereof.

A third group of QACs suitable for use according to the present disclosure is represented by formula (III): 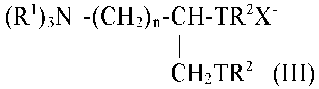 wherein each R¹ group is independently selected from C₁-C₄ alkyl, hydroxyalkyl or C₂-C₄ alkenyl groups; and wherein each R² group is independently selected from C₈-C₂₈ alkyl or alkenyl groups; and wherein n, T, and X are as defined above. Preferred materials of this second group include 1,2 bis[tallowoyloxy]-3-trimethylammonium propane chloride, 1,2 bis[hardened tallowoyloxy]-3-trimethylammonium propane chloride, 1,2-bis[oleoyloxy]-3 trimethylammonium propane chloride, and 1,2 bis[stearoyloxy]-3-trimethylammonium propane chloride. Such materials are described in US 4,137,180 (Lever Brothers). Preferably, these materials also comprise an amount of the corresponding mono-ester.

*Co-softeners.* Co-softeners, also referred to as co-softeners and fatty complexing agents may be used in fabric conditioner composition of the present disclosure. When employed, they are typically present at from 0.1 to 20% and particularly at from 0.1 to 5%, based on the total weight of the composition. Preferred co-softeners include fatty alcohols, fatty esters, and fatty N-oxides. Fatty esters that may be employed include fatty monoesters, such as glycerol monostearate, fatty sugar esters, such as those disclosed WO 01/46361 (Unilever).

In some embodiments, the compositions of the present disclosure may comprise a co-actives. Especially suitable fatty complexing agents include fatty alcohols and fatty acids. Of these, fatty alcohols are most preferred. Without being bound by theory it is believed that the fatty complexing material improves the viscosity profile of the composition by complexing with mono-ester component of the fabric conditioner material thereby providing a composition which has relatively higher levels of di-ester and tri-ester linked components. The di-ester and tri-ester linked components are more stable and do not affect initial viscosity as detrimentally as the mono-ester component. It is also believed that the higher levels of mono-ester linked component present in compositions comprising quaternary ammonium materials based on TEA may destabilize the composition through depletion flocculation. By using the co-active material to complex with the mono-ester linked component, depletion flocculation is significantly reduced. In other words, the co-active at the increased levels, as required by the present disclosure in some embodiments, "neutralizes" the mono-ester linked component of the quaternary ammonium material. This in situ di-ester generation from mono-ester and fatty alcohol also improves the softening of the composition.

*Silicone.* In some embodiments, the compositions of the present disclosure may further contain a silicone based fabric softening agent. Preferably the fabric softening silicone is a polydimethylsiloxane. The fabric softening silicones include but are not limited to 1) non-functionalized silicones such as polydimethylsiloxane (PDMS) or alkyl (or alkoxy) functional silicones; 2) functionalized silicones or copolymers with one or more different types of functional groups such as amino, phenyl, polyether, acrylate, silicon hydride, carboxylic acid, quaternized nitrogen, etc. Suitable silicones may be selected from polydialkylsiloxanes, preferably polydimethylsiloxane more preferably amino functionalised silicones; anionic silicones and carboxyl functionalized silicone. An amino silicone that may also be used, for example, Arristan 64, ex CHT or Wacker CT45E, ex Wacker.

In terms of silicone emulsions, the particle size can be in the range from about 1 nm to 100 microns and preferably from about 10 nm to about 10 microns including microemulsions ( < 150 nm), standard emulsions (about 200 nm to about 500 nm) and macroemulsions (about 1 micron to about 20 microns).

*Non-ionic surfactants.* In some embodiments, the compositions may further comprise a nonionic surfactant. Typically, these can be included for the purpose of stabilizing the compositions. Suitable nonionic surfactants include addition products of ethylene oxide with fatty alcohols, fatty acids, and fatty amines. Any of the alkoxylated materials of the particular type described hereinafter can be used as the nonionic surfactant. Suitable surfactants are substantially water soluble surfactants of the general formula (V): R-Y-(C₂H₄O)z-CH₂-CH₂-OH (V) where R is selected from the group consisting of primary, secondary and branched chain alkyl and/or acyl hydrocarbyl groups; primary, secondary and branched chain alkenyl hydrocarbyl groups; and primary, secondary and branched chain alkenyl-substituted phenolic hydrocarbyl groups; the hydrocarbyl groups having a chain length of from 8 to about 25, preferably 10 to 20, *e.g*., 14 to 18 carbon atoms. In the general formula for the ethoxylated nonionic surfactant, Y is typically: - O- , -C(O)O- , -C(O)N(R)- or -C(O)N(R)R in which R has the meaning given above for formula (V), or can be hydrogen; and Z is at least about 8, preferably at least about 10 or 11.

Preferably the nonionic surfactant has an HLB of from about 7 to about 20, more preferably from 10 to 18, *e.g*., 12 to 16. GENAPOL^{®} C200 (Clariant) based on coco chain and 20 EO groups is an example of a suitable nonionic surfactant. If present, the nonionic surfactant is present in an amount from 0.01 to 10%, more preferably 0.1 to 5 by weight, based on the total weight of the composition. LUTENSOL^{®} AT25 (BASF) based on coco chain and 25 EO groups is an example of a suitable non-ionic surfactant. Other suitable surfactants include RENEX^{®} 36 (Trideceth-6), ex Croda; TERGITOL^{®} 15-S3, ex Dow Chemical Co.; Dihydrol LT7, ex Thai Ethoxylate ltd; CREMOPHOR^{®} CO40, ex BASF and NEODOL^{®} 91-8, ex Shell.

*Cationic Polysaccharide.* In some embodiments, the compositions may further comprise at least one cationic polysaccharide. The cationic polysaccharide can be obtained by chemically modifying polysaccharides, generally natural polysaccharides. By such modification, cationic side groups can be introduced into the polysaccharide backbone The cationic polysaccharides are not limited to: cationic cellulose and derivatives thereof, cationic starch and derivatives thereof, cationic callose and derivatives thereof, cationic xylan and derivatives thereof, cationic mannan and derivatives thereof, cationic galactomannan and derivatives thereof, such as cationic guar and derivatives thereof. Cationic celluloses which are suitable include cellulose ethers comprising quaternary ammonium groups, cationic cellulose copolymers or celluloses grafted with a water-soluble quaternary ammonium monomer.

The cellulose ethers comprising quaternary ammonium groups are described in French patent 1,492,597 and in particular include the polymers sold under the names "JR" (JR 400, JR 125, JR 30M) or "LR" (LR 400, LR 30M) by the company Dow. These polymers are also defined in the CTFA dictionary as hydroxyethylcellulose quaternary ammoniums that have reacted with an epoxide substituted with a trimethylammonium group. Suitable cationic celluloses also include LR3000 KC from Solvay. The cationic cellulose copolymers or the celluloses grafted with a water-soluble quaternary ammonium monomer are described especially in patent US 4,131 ,576, such as hydroxyalkylcelluloses, for instance hydroxymethyl-, hydroxyethyl- or hydroxypropylcelluloses grafted especially with a methacryloyl-ethyltrimethylammonium, methacrylamidopropyltrimethylammonium or dimethyl-diallylammonium salt.

The commercial products corresponding to this definition are more particularly the products sold under the names CELQUAT^{®} L 200 and CELQUAT^{®} H 100 by Akzo Nobel. Cationic starches suitable for the present disclosure include the products sold under POLYGELO^{®} (cationic starches from Sigma), the products sold under SOFTGEL^{®}, AMYLOFAX^{®} and SOLVITOSE^{®} (cationic starches from Avebe), CATO from National Starch. Suitable cationic galactomannans can be those derived from Fenugreek Gum, Konjac Gum, Tara Gum, Cassia Gum or Guar Gum.

In some embodiments, the cationic polysaccharide of the present disclosure may have an average Molecular Weight (Mw) of between 100,000 daltons and 3,500,000 daltons, preferably between 100,000 daltons and 1,500,000 daltons, more preferably between 100,000 daltons and 1,000,000 daltons.

In some embodiments, the fabric conditioner composition of the present disclosure preferably comprises from 0.01 to 2 wt % of cationic polysaccharide based on the total weight of the composition. More preferably, 0.025 to 1 wt % of cationic polysaccharide based on the total weight of the composition. Most preferably, 0.04 to 0.8 wt % of cationic polysaccharide based on the total weight of the composition. In the context of the present application, the term "Degree of Substitution (DS)" of cationic polysaccharides, such as cationic guars, is the average number of hydroxyl groups substituted per sugar unit. DS may notably represent the number of the carboxymethyl groups per sugar unit. DS may be determined by titration.

The DS of the cationic polysaccharide is preferably in the range of 0.01 to 1, more preferably 0.05 to 1, most preferably 0.05 to 0.2. In the context of the present application, "Charge Density (CD)" of cationic polysaccharides, such as cationic guars, means the ratio of the number of positive charges on a monomeric unit of which a polymer is comprised to the molecular weight of said monomeric unit. CD of the cationic polysaccharide, such as the cationic guar, is preferably in the range of 0.1 to 3 (meq/gm), more preferably 0.1 to 2 (meq/gm), most preferably 0.1 to 1 (meq/gm).

*Non-ionic Polysaccharide.* In some embodiments, the fabric conditioner composition may further comprise at least one non-ionic polysaccharide. The nonionic polysaccharide can be a modified nonionic polysaccharide or a non-modified nonionic polysaccharide. The modified non-ionic polysaccharide may comprise hydroxyalkylation and/or esterification. In the context of the present disclosure, the level of modification of non-ionic polysaccharides can be characterized by Molar Substitution (MS), which means the average number of moles of substituents, such as hydroxypropyl groups, per mole of the monosaccharide unit. MS can be determined by the Zeisel-GC method, notably based on the following literature reference: Hodges, et al. (1979) Anal. Chem. 51(13). Preferably, the MS of the modified nonionic polysaccharide is in the range of 0 to 3, more preferably 0.1 to 3 and most preferably 0.1 to 2.

In some embodiments, the nonionic polysaccharide of the present disclosure may be especially chosen from glucans, modified or non-modified starches (such as those derived, for example, from cereals, for instance wheat, corn or rice, from vegetables, for instance yellow pea, and tubers, for instance potato or cassava), amylose, amylopectin, glycogen, dextrans, celluloses and derivatives thereof (methylcelluloses, hydroxyalkylcelluloses, ethylhydroxyethylcelluloses), mannans, xylans, lignins, arabans, galactans, galacturonans, chitin, chitosans, glucuronoxylans, arabinoxylans, xyloglucans, glucomannans, pectic acids and pectins, arabinogalactans, carrageenans, agars, gum Arabics, gum tragacanths, ghatti gums, karaya gums, carob gums, galactomannans suchas guars and nonionic derivatives thereof (hydroxypropyl guar), and mixtures thereof.

Among the celluloses that can be especially used are hydroxyethylcelluloses and hydroxypropylcelluloses. Suitable non-limiting examples include products sold under the trade names KLUCEL^{®} EF, KLUCEL^{®} H, KLUCEL^{®} LHF, KLUCEL^{®} MF and KLUCEL^{®} G by Aqualon, and CELLOSIZE^{®} Polymer PCG-10 by Amerchol, and HEC, HPMC K200, HPMC K35M by Ashland.

In some embodiments, the fabric conditioner composition of the present disclosure preferably comprises from 0.01 to 2 wt % of non-ionic polysaccharide based on the total weight of the composition. More preferably, 0.025 to 1 wt % of non-ionic polysaccharide based on the total weight of the composition. Most preferably, 0.04 to 0.8 wt % of non-ionic polysaccharide based on the total weight of the composition. Preferably the fabric conditioning composition comprises combined weight of the cationic polysaccharide and non-ionic polysaccharide of 0.02 to 4 wt %, more preferably 0.05 to 2 wt % and most preferably 0.08 to 1.6 wt %. Preferably the ratio of the weight of the cationic polysaccharide in the composition and the weight of the nonionic polysaccharide in the composition is between 1:10 and 10:1, more preferably, between 1:3 and 3:1.

In a preferred embodiment, the cationic polysaccharide and non-ionic polysaccharide are mixed prior to addition to the fabric conditioner composition. Preferably the mix is prepared as a suspension in water. Preferably, the ratio of the weight of the quaternary ammonium compound in the composition and the total weight of the cationic polysaccharide and the nonionic polysaccharide in the composition is between 100:1 and 2:1, more preferably, between 30:1 and 5:1.

*Water.* In some embodiments, the fabric conditioner composition of the present disclosure comprises water. The compositions are rinse-added softening compositions suitable for use in a laundry process. The compositions are pourable liquids. The liquid compositions have a pH ranging from about 2.0 to about 7, preferably from about 2 to about 4, more preferably from about 2.5 to about 3.5. The compositions may also contain pH modifiers preferably hydrochloric acid, lactic acid or sodium hydroxide. The composition is preferably a ready-to-use liquid comprising an aqueous phase. The aqueous phase may comprise water-soluble species, such as mineral salts or short chain (C₁-C₄) alcohols. The composition is preferably for use in the rinse cycle of a home textile laundering operation, where, it may be added directly in an undiluted state to a washing machine, *e.g*., through a dispenser drawer or, for a top-loading washing machine, directly into the drum. The compositions may also be used in a domestic hand-washing laundry operation.

The fabric conditioner composition may typically be made by combining a melt comprising the fabric softening agent with an aqueous phase. The polymer may be combined with the water phase, or it may be post dosed into the composition after combination of the melt and water phase. A preferred method of preparation is as follows:
1. Heat water to about 40 to 50°C, preferably above 45°C.
2. Add the rheology modifiers to the water slowly, preferably over about 1 minute with stirring.
3. Mix thoroughly, preferably from 1 to 10 minutes.
4. Add any minor ingredients, such as antifoams, sequestrants and preservatives.
5. Melt the softening active and optional fatty alcohol together to form a co-melt.
6. Add the co-melt to the heated water.
7. Add acid to the preferred pH, if required.
8. Add dyes and perfumes.
9. Cool.

B) Liquid dish detergents such as those described in US 6,069,122 and US 5,990,065.

C) Automatic Dish Detergents such as those described in US 6,020,294, US 6,017,871, US 5,968,881, US 5,962,386, US 5,939,373, US 5,914,307, US 5,902,781, US 5,705,464, US 5,703,034, US 5,703,030, US 5,679,630, US 5,597,936, US 5,581,005, US 5,559,261, US 4,515,705, US 5,169,552, and US 4,714,562.

D) All-purpose cleaners including bucket dilutable cleaners and toilet cleaners, bathroom cleaners, bath tissue, rug deodorizers, candles (*e.g*., scented candles), room deodorizers, floor cleaners, disinfectants, window cleaners, garbage bags/ trash can liners, air fresheners (*e.g*., room deodorizer, car deodorizer, sprays, scent oil air freshener, automatic spray air freshener, and neutralizing gel beads), moisture absorber, household devices (*e.g*., paper towels and disposable wipes), and moth balls/traps/cakes.

E) Personal care products: cosmetic or pharmaceutical preparations. More specifically personal cleansers (*e.g*., bar soaps, body washes, and shower gels), in-shower conditioner, sunscreen (*e.g*., sprays, lotions and sticks), insect repellents, hand sanitizers, anti-inflammatory (*e.g*., balms, ointments and sprays), antibacterial (*e.g*., ointments and creams), sensates, deodorants and antiperspirants (including aerosol, pump spray and wax based), lotions, body powder and foot powder, body mist or body spray, shave cream and male groom products, bath soak, exfoliating scrub.

F) Hair Care products. More specifically, shampoos (liquid and dry powder), hair conditioners (rinse-out conditioners, leave-in conditioners, and cleansing conditioners), hair rinses, hair refreshers, hair perfumes, hair straightening products, hair styling products, hair fixative and styling aids, hair combing creams, hair wax, hair foam, hair gel, non-aerosol pump spray, hair bleaches, dyes and colorants, perming agents, and hair wipes.

In particular aspects, the core-shell microcapsule slurry of this disclosure is of use in improving a freshness impression to a fabric. Accordingly, in certain aspects, the microcapsules of the present disclosure are included in a fabric conditioner or softener having a pH of from 2 to 4, preferably a pH of from 2.5 to 3.5.

Many aspects and embodiments have been described above and are merely exemplary and not limiting. After reading this specification, skilled artisans appreciate that other aspects and embodiments are possible without departing from the scope of the invention.

### EXAMPLES

The following non-limiting examples are provided to further illustrate the present invention and are not to be construed as limitations of the invention, as many variations of the present invention are possible without departing from its spirit or scope.

### General

Caprylic/capric triglyceride used in the Examples is the commercial product under the tradename NEOBEE^{®} M-5 from Stepan in Chicago, IL. The polyisocyanate used in the Examples is trimethylol propane-adduct of xylylene diisocyanate commercially available under the tradename TAKENATE^{®} D110N, Mitsue Chemicals Inc., Japan. A person of ordinary skill in the art appreciates that some polyisocyanate commercial products is a solution of polyisocyanate in a solvent. The polyisocyanate (e.g., TAKENATE^{®} D110N) amount indicated in this disclosure means the amount of polyisocyanate itself, that is, the amount of the solvent is excluded.

### Example 1: Synthesis of Reference Microcapsules

As described in Example 1 of US 2012/0093899, melamine formaldehyde capsules were prepared. Briefly, 80 parts by weight of Helion fragrance (International Flavors & Fragrance Inc., Union Beach, NJ) was admixed with 20 parts by weight of caprylic/capric triglyceride solvent thereby forming a fragrance/solvent composition. The uncoated capsules were prepared by creating a polymeric wall to encapsulate fragrance/solvent composition droplets. A copolymer of acrylamide and acrylic acid (sold under the tradename ALCAPSOL^{®} 200) was first dispersed in water together with a methylated melamine formaldehyde resin (sold under the tradename CYMEL^{®} 385). These two components were allowed to react under acidic conditions for at least one hour.

The fragrance/solvent composition was then added to the wall polymer solution and droplets of the desired size were achieved by high shear homogenization. For the microcapsule slurry, curing of the polymeric layer around the fragrance/solvent composition droplets was carried out at 125 °C. After cooling to room temperature, ethylene urea was added into the microcapsule slurry. Additionally, a rheology modifier and a preservative were added. The pH was adjusted using NaOH. The components of the slurry are listed in Table 1. The slurry contained an overall fragrance load of 28.0%.

**Table 1 - Composition of the Slurry**

| **Ingredient** | **Amount (g)** | **Weight %** |
|---|---|---|
| Fragrance | 182 | 28 |
| Caprylic/capric triglyceride | 45.5 | 7 |
| Copolymer of acrylamide and acrylic acid | 73.9 | 11.4 |
| Methylated melamine formaldehyde resin | 9.9 | 1.5 |
| Ethylene Urea | 13.3 | 2.0 |
| Acetic Acid | 2.4 | 0.4 |
| Sodium hydroxide | 1.1 | 0.2 |
| Acrylates copolymer¹ | 6.5 | 1 |
| 1,2-Benzisothiazolin-3-one ² | 0.7 | 0.1 |
| Water | 314.8 | 48.4 |
| **Total** | **650** | **100%** |

| | | |
|---|---|---|
| ¹ available as ACULYN^{®} 33A. ² available as PROXEL^{®} GXL. | | |

### Example 2: Preparation of Polyisocyanate Microcapsules in the Presence of Denatured Pea Protein, Modified Starch/Polystyrene Sulfonate, and Sodium Salt

An oil phase was prepared by mixing 80 parts by weight of Helion fragrance with 20 parts by weight of caprylic/capric triglyceride solvent thereby forming a fragrance/solvent composition. An aqueous phase was prepared by dispersing pea protein powder (15.4 weight %) in water. Guanidine carbonate, as a denaturing agent, was added and pH was adjusted to 5 using citric acid. These components were allowed to react for 15 minutes.

Modified starch sold under the tradename PURITY GUM^{®} Ultra (Ingredion, Westchester, IL) and high molecular weight polystyrene sulfonate, sodium salt sold under the tradename FLEXAN^{®} II were then added to the aqueous phase as emulsifiers and the mixture was allowed to mix for 15 minutes. Tanal-02 (a high molecular weight general purpose hydrolysable tannin; Ajinomoto Natural Specialties, Tokyo, Japan) was subsequently added to the aqueous phase.

A polyisocyanate was added to the oil phase at 5 weight %. The oil phase was then emulsified into the aqueous phase to form an oil-in-water emulsion (i.e., microcapsule slurry) under a shearing rate of 7400 revolutions per minute ("RPM") for 3 minutes. For the microcapsule slurry, curing of the polymeric layer (i.e., shell) around the fragrance/solvent composition droplets was carried out at 55 °C for 3.5 hours and 80 °C for 30 minutes. Subsequently, a rheology modifier and a preservative (1,2-benzisothiazolin-3-one) were added. The components of the slurry are listed in Table 2. The slurry contained an overall fragrance load of 31.2%.

**Table 2 - Composition of the Slurry**

| **Ingredient** | **Amount (g)** | **Weight %** |
|---|---|---|
| Fragrance | 187.2 | 31.2 |
| Caprylic/capric triglyceride | 46.8 | 7.8 |
| Pea protein | 17.5 | 2.9 |
| Guanidine Carbonate | 7.6 | 1.3 |
| Citric Acid | 7.3 | 1.2 |
| High molecular weight polystyrene sulfonate, sodium salt ¹ | 2.9 | 0.5 |
| Modified Starch ² | 5.9 | 1.0 |
| Trimethylol propane-adduct of xylylene diisocyanate ³ | 5.85 | 1 |
| Tanal-02 | 2.9 | 0.5 |
| Xanthan gum | 0.5 | 0.08 |
| 1,2-Benzisothiazolin-3-one ⁴ | 0.7 | 0.1 |
| Water | 309 | 51.5 |
| **Total** | **600** | **100%** |

| | | |
|---|---|---|
| ¹ available as FLEXAN^{®} II. ² available as PURITY GUM^{®} Ultra. ³ available as TAKENATE^{®} D110N. ⁴ available as PROXEL^{®} GXL. | | |

### Example 3: Preparation of Polyisocyanate Microcapsules in the Presence of Denatured Pea Protein, Modified Starch/Polystyrene Sulfonate, Sodium Salt at pH 4 and a Curing Temperature of 65 °C

The general procedure of Example 2 was followed with the following changes: the pH of the aqueous phase was adjusted to 4 instead of 5 and the curing was carried out at 65 °C for 4 hours. The components of the slurry are listed in Table 3. The slurry contained an overall fragrance load of 31.2%.

**Table 3 - Composition of the Slurry**

| **Ingredient** | **Amount (g)** | **Weight %** |
|---|---|---|
| Fragrance | 187.2 | 31.2 |
| Caprylic/capric triglyceride | 46.8 | 7.8 |
| Pea protein | 17.5 | 2.9 |
| Guanidine Carbonate | 7.6 | 1.3 |
| Citric Acid | 14.0 | 2.3 |
| High molecular weight polystyrene sulfonate, sodium salt ¹ | 2.9 | 0.5 |
| Modified Starch ² | 5.9 | 1.0 |
| Trimethylol propane-adduct of xylylene diisocyanate ³ | 5.85 | 1 |
| Tanal-02 | 2.9 | 0.5 |
| Xanthan gum | 0.5 | 0.08 |
| 1,2-Benzisothiazolin-3-one *⁴* | 0.7 | 0.1 |
| Water | 302.3 | 50.4 |
| **Total** | **600** | **100%** |

| | | |
|---|---|---|
| *¹* available as FLEXAN^{®} II. *²* available as PURITY GUM^{®} Ultra. *³* available as TAKENATE^{®} D110N. *⁴* available as PROXEL^{®} GXL. | | |

### Example 4: Preparation of Microcapsules with Reduced Levels of Denatured Pea Protein

The general procedure of Example 3 was carried out with a reduced concentration of pea protein. The components of the slurry are listed in Table 4. The slurry contained an overall fragrance load of 31.2%.

**Table 4 - Composition of the Slurry**

| **Ingredient** | **Amount (g)** | **Weight %** |
|---|---|---|
| Fragrance | 187.2 | 31.2 |
| Caprylic/capric triglyceride | 46.8 | 7.8 |
| Pea protein | 11 | 1.8 |
| Guanidine Carbonate | 7.6 | 1.3 |
| Citric Acid | 14.0 | 2.3 |
| High molecular weight polystyrene sulfonate, sodium salt ¹ | 2.9 | 0.5 |
| Modified Starch ² | 5.9 | 1.0 |
| Trimethylol propane-adduct of xylylene diisocyanate ³ | 5.85 | 1 |
| Tanal-02 | 2.9 | 0.5 |
| Xanthan gum | 0.5 | 0.08 |
| 1,2-Benzisothiazolin-3-one ⁴ | 0.7 | 0.1 |
| Water | 308.8 | 51.5 |
| **Total** | **600** | **100%** |

| | | |
|---|---|---|
| *¹* available as FLEXAN^{®} II. *²* available as PURITY GUM^{®} Ultra. *³* available as TAKENATE^{®} D110N. *⁴* available as PROXEL^{®} GXL. | | |

### Example 5: Preparation of Microcapsules Under Lower pH Conditions

The general procedure of Example 3 was carried out except that the pH of the aqueous phase was adjusted to 3 instead of 4. The components of the slurry are listed in Table 5. The slurry contained an overall fragrance load of 30.3%.

**Table 5 - Composition of the Slurry**

| **Ingredient** | **Amount (g)** | **Weight %** |
|---|---|---|
| Fragrance | 187.2 | 30.3 |
| Caprylic/capric triglyceride | 46.8 | 7.6 |
| Pea protein | 17.5 | 2.8 |
| Guanidine Carbonate | 7.6 | 1.2 |
| Citric Acid | 21.5 | 3.5 |
| High molecular weight polystyrene sulfonate, sodium salt *¹* | 2.9 | 0.5 |
| Modified Starch *²* | 5.9 | 1.0 |
| Trimethylol propane-adduct of xylylene diisocyanate *³* | 5.85 | 0.9 |
| Tanal-02 | 2.9 | 0.5 |
| Xanthan gum | 0.5 | 0.08 |
| 1,2-Benzisothiazolin-3-one *⁴* | 0.7 | 0.1 |
| Water | 311.7 | 50.5 |
| **Total** | **616.9** | **100%** |

| | | |
|---|---|---|
| *¹* available as FLEXAN^{®} II. *²* available as PURITY GUM^{®} Ultra. *³* available as TAKENATE^{®} D110N. *⁴* available as PROXEL^{®} GXL. | | |

### Example 6: Preparation of Microcapsules with pH Adjusted by Using Phosphoric Acid

The general procedure of Example 3 was carried out except that the pH of the aqueous phase was adjusted by using phosphoric acid instead of citric acid. The components of the slurry are listed in Table 6. The slurry contained an overall fragrance load of 32.2%.

**Table 6 - Composition of the Slurry**

| **Ingredient** | **Amount (g)** | **Weight %** |
|---|---|---|
| Fragrance | 187.2 | 32.2 |
| Caprylic/capric triglyceride | 46.8 | 8.1 |
| Pea protein | 17.5 | 3.0 |
| Guanidine Carbonate | 7.6 | 1.3 |
| Phosphoric acid | 9.4 | 1.5 |
| High molecular weight polystyrene sulfonate, sodium salt ¹ | 2.9 | 0.5 |
| Modified Starch ² | 5.9 | 1.0 |
| Trimethylol propane-adduct of xylylene diisocyanate ³ | 5.85 | 1 |
| Tanal-02 | 2.9 | 0.5 |
| Xanthan gum | 0.5 | 0.08 |
| 1,2-Benzisothiazolin-3-one ⁴ | 0.7 | 0.1 |
| Water | 302.3 | 52 |
| **Total** | **581** | **100%** |

| | | |
|---|---|---|
| *¹* available as FLEXAN^{®} II. *²* available as PURITY GUM^{®} Ultra. *³* available as TAKENATE^{®} D110N. ⁴ available as PROXEL^{®} GXL. | | |

### Example 7: Preparation of Microcapsules with Increased Fragrance Load

The general procedure of Example 3 was followed with a reduced amount of water. The components of the slurry are listed in Table 7. The slurry contained an overall fragrance load of 34.6%.

**Table 7 - Composition of the Slurry**

| **Ingredient** | **Amount (g)** | **Weight %** |
|---|---|---|
| Fragrance | 187.2 | 34.6 |
| Caprylic/capric triglyceride | 46.8 | 8 |
| Pea protein | 17.5 | 3.0 |
| Guanidine Carbonate | 7.6 | 1.3 |
| Citric Acid | 5.7 | 1.0 |
| High molecular weight polystyrene sulfonate, sodium salt ¹ | 2.9 | 0.5 |
| Modified Starch ² | 5.9 | 1.0 |
| Trimethylol propane-adduct of xylylene diisocyanate ³ | 5.85 | 2.0 |
| Tanal-02 | 5.8 | 1.0 |
| Xanthan gum | 0.5 | 0.09 |
| 1,2-Benzisothiazolin-3-one ⁴ | 0.7 | 0.1 |
| Water | 296.3 | 51.0 |
| **Total** | **581** | **100%** |

| | | |
|---|---|---|
| *¹* available as FLEXAN^{®} II. *²* available as PURITY GUM^{®} Ultra. *³* available as TAKENATE^{®} D110N. *⁴* available as PROXEL^{®} GXL. | | |

### Example 8: Preparation of Microcapsules with a Higher Concentration of Surfactant

The general procedure of Example 3 was followed with a greater amount of surfactant. The components of the slurry are listed in Table 8. The slurry contained an overall fragrance load of 28.6%.

**Table 8 - Composition of the Slurry**

| **Ingredient** | **Amount (g)** | **Weight %** |
|---|---|---|
| Fragrance | 187.2 | 28.6 |
| Caprylic/capric triglyceride | 46.8 | 7.2 |
| Pea protein | 17.5 | 2.7 |
| Guanidine Carbonate | 7.6 | 1.3 |
| Citric Acid | 5.2 | 0.8 |
| High molecular weight polystyrene sulfonate, sodium salt *¹* | 4.4 | 0.7 |
| Modified Starch *²* | 8.9 | 1.4 |
| Trimethylol propane-adduct of xylylene diisocyanate *³* | 5.85 | 0.9 |
| Tanal-02 | 2.9 | 0.4 |
| Xanthan gum | 0.5 | 0.08 |
| 1,2-Benzisothiazolin-3-one *⁴* | 0.7 | 0.1 |
| Water | 320.05 | 53.3 |
| **Total** | **600** | **100%** |

| | | |
|---|---|---|
| *¹* available as FLEXAN^{®} II. *²* available as PURITY GUM^{®} Ultra. *³* available as TAKENATE^{®} D 110N. *⁴* available as PROXEL^{®} GXL. | | |

### Example 9: Microcapsules Prepared with Denatured Pea Protein and Gum Arabic

An oil phase was prepared by mixing 80 parts by weight of Helion fragrance with 20 parts by weight of caprylic/capric triglyceride solvent thereby forming a fragrance/solvent composition. A polyisocyanate was added to the oil phase at 5 weight %.

An aqueous phase was prepared by dispersing 12.43 grams of pea protein powder in 124 grams of water and adjusting the pH to 9-9.5 using 0.3 grams of 25% sodium hydroxide solution. To facilitate dissolution and inhibit aggregation of the pea protein isolate (Liu, et al. (2010) Food Res. Internatl. 43:489-495), 85 grams of a gum Arabic Instant AA (Nexira, Somerville, NJ; 10% solution) was included as a hydrocolloid. The aqueous mixture was high sheared for 20 seconds at 7400 rpm. High molecular weight polystyrene sulfonate, sodium salt sold under the tradename FLEXAN^{®} II (15 grams of a 10% solution) was added and the resulting aqueous mixture was high sheared for 20 seconds at 7400 rpm. In a separate beaker, 38 grams guanidine carbonate solution (20%) was adjusted to pH of 4 using 31 grams of a 50% solution of citric acid and the resulting solution was allowed to foam out. The resulting guanidine citrate solution was added to the aqueous pea protein/gum Arabic mixture and allowed to react for 15 minutes at room temperature. Forty-eight grams of a 1% solution of xanthan gum was subsequently added to the aqueous phase followed by 10 grams of a 30% solution of Tanal-02.

The oil phase was then emulsified into the aqueous phase to form an oil-in-water emulsion (i.e., microcapsule slurry) under a shearing rate of 7400 rpm for 3 minutes. For the microcapsule slurry, curing of the polymeric layer (i.e., shell) around the fragrance/solvent composition droplets was carried out at 65 °C for 4 hours. Additionally, a preservative was added to the slurry. The components of the slurry are listed in Table 9. The slurry contained an overall fragrance load of 31.2%.

**Table 9 - Composition of the Slurry**

| **Ingredient** | **Amount (g)** | **Weight %** |
|---|---|---|
| Fragrance | 187.2 | 31.2 |
| Caprylic/capric triglyceride | 46.8 | 7.8 |
| Pea protein | 12.4 | 2 |
| Guanidine Carbonate | 7.6 | 1.3 |
| Citric Acid | 15.5 | 2.5 |
| High molecular weight polystyrene sulfonate, sodium salt ¹ | 1.5 | 0.25 |
| Gum Arabic | 8.5 | 1.4 |
| Trimethylol propane-adduct of xylylene diisocyanate ² | 5.85 | 0.97 |
| Tanal-02 | 3 | 0.5 |
| Xanthan gum | 0.5 | 0.08 |
| 1,2-Benzisothiazolin-3-one ³ | 0.7 | 0.1 |
| Water | 318.05 | 53 |
| **Total** | **600** | **100** |

| | | |
|---|---|---|
| *¹* available as FLEXAN^{®} II. *²* available as TAKENATE^{®} D 110N. *⁴* available as PROXEL^{®} GXL. | | |

Same microcapsule slurry was prepared except without gum Arabic. However, without gum Arabic the emulsion completely failed and microcapsule was not formed. This demonstrated that a polyisocyanate/pea protein microcapsule could not be formed in the absence of gum Arabic.

### Example 10: Fabric Conditioner Samples Containing Microcapsules

An un-fragranced model fabric conditioner having a 10% hole in the formulation was used to allow for water and microcapsules to be added. Microcapsules as described in Examples 1-3 were pre-mixed with water respectively and then added to the model fabric conditioner. The resulting samples were homogenized using an overhead agitator at 300 rpm. The finished fabric conditioner samples contained 0.2% neat oil equivalent resulting in 0.65 weight % encapsulated fragrance for the microcapsules in Examples 2 and 3 and 0.72 weight % encapsulated fragrance for the reference microcapsules in Example 1.

Thirty-five grams of finished fabric conditioners containing the above-referenced dosage of microcapsules were added to a front load Miele Professional PW 6065 Vario washing machine. The wash load contained 2.2 kg of laundry including eight big towels, two t-shirts, two pillow cases, two dish towels, and two mini-towels for evaluation. The washing temperature was set to 40 °C with 15.5 L of water used for the main wash and 34 L of total water for two rinses. The total washing cycle was 60 minutes. Some towels were kept for damp evaluation and the rest were line dried at room temperature for dry evaluation.

Randomly selected damp samples were evaluated by several experts using the intensity scale 0-5, where 0 is "no performance" and 5 is "strong performance." The evaluation was performed "blind", such that each sample had a randomly allocated number. The dry evaluation was performed the day following the damp and was performed by the same experts using the same intensity scale of 0-5. Sensory scores were recorded before and after each of the randomly selected cloths (contained in a separate polyethylene bag) was gently handled. The results of these analyses are presented in Table 10.

Example 3, which is the pea protein/isocyanate microcapsule with low pH and low curing temperature, performed better by providing a strong fragrance burst during dry evaluation (post-handling) than both melamine formaldehyde microcapsule (Example 1) and pea protein/isocyanate microcapsule with high pH and high curing temperature (Example 2). Furthermore, Example 3 microcapsules demonstrated that they survived the damp stage on cloth, even though they were relatively weak compared to the Example 2 microcapsules. Moreover, Example 3 microcapsules demonstrated improved processability, no aggregate formation and improved slurry color when compared to Examples 1 and 2 microcapsules.

**Table 10 - Sensory Evaluation Results**

| **Exemplary Microcapsule** | **Damp Evaluation** | **Dry Evaluation** | |
|---|---|---|---|
| | | **Pre-Handling** | **Post-Handling** |
| 1 | 3.56 | 3.19 | 4.05 |
| 2 | 3.88 | 3.36 | 3.74 |
| 3 | 3.60 | 3.29 | 4.13 |

### Example 11: Analytical Evaluation of Different Microcapsules

Characteristics including fragrance load, encapsulation efficiency, free oil, viscosity and size of the microcapsules produced in Examples 1, 3, 4, 5, 6 and 9 were determined. The results of these analyses are presented in Table 11.

**Table 11 - Analytical Evaluation Results**

| **Exemplary Microcapsule** | **Fr. Load *¹* %** | **EE *²* %** | **Free Oil %** | **Viscosity *³* (cps; 21 s-1)** | **PSD *⁴* (Mean/Mode)** |
|---|---|---|---|---|---|
| 1 | 28 | >95 | 0.39 | 625 | 6.7/5.4 |
| 3 | 31.2 | >95 | 0.3-1.9 | 574 | 22.1/15.6 |
| 4 | 31.2 | >95 | 0.2 | 605 | 34.5/16.2 |
| 5 | 30.3 | >95 | 0.4 | 495 | 24.5/18.9 |
| 6 | 32.2 | >95 | 0.2 | 530 | 22.9/16.7 |
| 9 | 31.2 | >95 | 0.18 | 357 | 23.3/24.3 |

| | | | | | |
|---|---|---|---|---|---|
| *¹* Fr. Load = Fragrance Load. *²* EE = Encapsulation Efficiency. *³* Viscosity was measured on a hake plate rheometer using 5, 21, and 64 sec shear rates. *⁴* PSD = particle size distribution. Tbm, to be measured. | | | | | |

In addition, wall strength was determined for microcapsules prepared in Example 9 as compared to whey microcapsules prepared in accordance with Example 7 in WO 2020/131875 A2 or microcapsules prepared in accordance with Example 2. This analysis, presented in FIG. 1, indicates that the choice of protein had a smaller influence on the wall strength and flexibility of the microcapsules. The pH and cure profile have a stronger effect on the wall strength while maintaining the flexibility of the wall (deformation). This combination allows for the minimal damp performance but very strong burst with minimal friction on the dry stages. The wall strength is so weak that minimal energy breaks the wall but the flexibility is sufficient to survive the wash cycle in a EU washing machine and the damp stage on cloth. Even though the polyisocyanate/pea protein-based microcapsules are relatively weak compared to the whey microcapsules or melamine formaldehyde microcapsules, polyisocyanate/pea protein-based microcapsules have good stability in product and processability of the slurry is maintained.

### Example 12: Malodor Absorption Capabilities

To test the malodor absorption capabilities of the microcapsules disclosed herein, diethyl phthalate and caprylic/capric triglyceride solvent were encapsulated according to the methods presented in Example 1 (melamine formaldehyde) and Example 9 (polyisocyanate microcapsule prepared with pea protein and gum Arabic) respectively to generate odorless microcapsules.

The microcapsules were exposed to malodor and the reduction of the malodor concentration was measured via headspace analysis. More specifically, 100 grams of 1.5% malodor solution was placed into a jar and allowed to equilibrate for 30 minutes. A towel was "activated" by rubbing the towel five times with a tongue depressor on a side marked with an "X". The "activated" towel, with "X" side up, was placed in a second jar (16 oz.) fitted with a septa injection lid. With a 100 mL gas tight syringe, 100 mL of malodor vapor was transferred into the second jar containing the towel sample. The towel sample was stored for 1.5 hours and headspace was subsequently analyzed using a SKC pump with 150 ml/min flow, sampling for 10 minutes on to a tenax tube.

The results of this analysis (Table 12) indicate that polyisocyanate microcapsules prepared with pea protein and gum Arabic have malodor absorption capabilities comparable to melamine formaldehyde microcapsules.

**Table 12 - Malodor Absorption Evaluation Results**

| **Malodor** | **Mean Area** | | |
|---|---|---|---|
| | **Blank** | **Example 1** | **Example 9** |
| Iso valeraldehyde | 2805176984 | 1640184599 | 1370641528 |
| Acetyl methyl carbinol | 1990840968 | 302635768 | 168033889.5 |
| Methyl pyrazine | 1800621600 | 318617731 | 176231698 |
| Heptanal | 935200716 | 558749725.5 | 545558189 |

### Example 13: Microcapsules Prepared with Oils Containing a High Concentration of Natural Components

The performance of microcapsules encapsulating natural fragrances (*i.e.,* extracts from plants or distillation products) or naturally derived fragrances (*i.e.,* natural fragrances that have been chemically modified) was also assessed (Table 13). These microcapsules were prepared in accordance with the method described in Example 9.

**Table 13 - Evaluation Results**

| **Fragrance** | **% Naturals & Naturally derived** | **Free Oil (%)** | **Viscosity (cps)** | **Performance** | | **Leakage at 5 weeks (%)** |
|---|---|---|---|---|---|---|
| | | | **(5 s-1)** | at fresh | at 4 weeks | |
| | | | **(21 s-1)** | | | |
| | | | **(106 s-1)** | | | |
| Tea Leaves | 23.5% (15.5% Essential Oils) | 0.25 | 476 | ++ | ++ | <10 |
| | | | 293 | | | |
| | | | 214 | | | |
| Apple 2 | 17% (3.5% Essential Oils) | 0.22 | 584 | ++ | ++ | <10 |
| | | | 358 | | | |
| | | | 273 | | | |
| Bamboo 2 | (3.2% Essential Oils) | 0.44 | 569 | ++ | ++ | <10 |
| | | | 315 | | | |
| | | | 175 | | | |
| Clean Linen | 18% (8% Essential Oils) | 0.23 | 451 | ++ | ++ | <10 |
| | | | 270 | | | |
| | | | 190 | | | |
| Rose | 15% (5% Essential oils) | 0.52 | 476 | ++ | ++ | <10 |
| | | | 281 | | | |
| | | | 197 | | | |
| Rose litchi | 14.5% (3% Essential Oils) | 0.35 | 465 | ++ | ++ | <10 |
| | | | 265 | | | |
| | | | 174 | | | |
| Mango | 69.55% (16.68% Essential Oils) | 0.46 | 462 | ++ | - | <10 |
| | | | 299 | | | |
| | | | 231 | | | |
| Watermelon | 22.3% naturally derived/ 3.7% natural | 0.41 | 479 | + | +/- | 16 |
| | | | 308 | | | |
| | | | 239 | | | |
| Lavender Blackberry | 51.09% (9.82% Essential Oils) | 0.48 | 513 | - | n/a | n/a |
| | | | 331 | | | |
| | | | 254 | | | |
| Lavender | 100% (45% Essential Oils) | 0.35 | 536 | ++ | ++ | <10 |
| | | | 325 | | | |
| | | | 245 | | | |
| Tubereuz | 13.3% naturals & 23% naturally derived | 0.67 | 484 | ++ | ++ | 14 |
| | | | 284 | | | |
| | | | 196 | | | |
| Eau d'Oranger | 45% naturally derived & 10.3% natural | 0.37 | 516 | + | + | <10 |
| | | | 304 | | | |
| | | | 214 | | | |
| Citrus Spicy | 100% (74.58% Essential Oils) | 0.52 | 502 | - | n/a | n/a |
| | | | 283 | | | |
| | | | 173 | | | |
| Xmas Tree | 99% (23.5% Essential Oils) | 0.52 | 704 | + | + | n/a |
| | | | 472 | | | |
| | | | 388 | | | |

| | | | | | | |
|---|---|---|---|---|---|---|
| "++" represents excellent performance burst and hedonics on dry. "+" represents good performance with burst on dry and hedonics. "-" represents poor performance on dry and hedonics. n/a, not available. | | | | | | |

Leakage of fragrance from the microcapsules prepared in accordance with the method described in Example 9 was evaluated after storage at 37 °C in fabric conditioner. The results of this analysis (Table 14) show stable encapsulation of oils containing high amounts of natural extracts and essential oils.

The performance of polyisocyanate microcapsules prepared in accordance with the method described in Example 9 were compared to melamine formaldehyde microcapsules (Example 1) at damp, dry pre, dry GH (gentle handling) and dry post stages. "Dry pre" means the stage after drying but before the folding of the cloth. "Dry GH" means the stage of folding the cloth twice, followed by the evaluation of the cloth by the panelists. "Dry post" means the stage with vigorous application of mechanical force using both hands to rub the cloth at least once to rupture the testing microcapsule and then evaluate for signs of released fragrance. Fragrance intensity was determined on a scale of 0-5, where 0 is no performance and 5 is maximum. Strength and hedonics were assessed by perfumers and scent design managers.

**Table 14 - Evaluation Results**

| **Fragrance** | **Microcapsule** | **Damp** | **Dry Pre** | **Dry GH** | **Dry Post** |
|---|---|---|---|---|---|
| Tea Leaves | Ex. 9 | ++ | ++ | ++ | ++ |
| | Ex. 1 (ref) | + | +/- | +/- | +/- |
| Apple 2 | Ex. 9 | ++ | + | + | + |
| | Ex. 1 (ref)^{∗} | ++ | ++ | ++ | ++ |
| Lavender | Ex. 9 | ++ | ++ | ++ | ++ |
| | Ex. 1 (ref) | ++^ | ++^ | ++^ | ++^ |

| | | | | | |
|---|---|---|---|---|---|
| ^{∗}, Failed, due to high viscosity in process. ++, stable performance and hedonics. +, stable performance, but less character. +/-, less performance and character. ^, Difference in release profile, but acceptable. | | | | | |

The expert evaluation with scent design managers and perfumers indicated that the hedonics was stable for microcapsules produced by the method described in Example 9 for the oils containing high level of naturals (Table 14). By comparison, melamine formaldehyde microcapsules did not show good encapsulation or stable performance overtime in the product.

### Example 14: Microcapsules Containing Low logP Fragrances

The stability and performance of microcapsules encapsulating low logP fragrances were also assessed (Tables 16 and 17). These testing microcapsules were prepared in accordance with the method described in Example 1 (melamine formaldehyde capsule) and Example 9 (polyisocyanate capsule) respectively, except that the fragrance composition to be encapsulated in this Example is a mixture of a fragrance base and low logP fragrance(s) indicated in Tables 16 and 17. Ethyl vanillin has logP value of 1.55, benzaldehyde has logP value of 1.70, cinnamaldehyde has logP value of 1.82, oxyphenylon has logP value of 1.48, veltol has logP value of 0.50, coumarin has logP value of 1.51, and aubepine has logP value of 2.20. The fragrance base has composition shown below in Table 15. Ingredients of the fragrance base have logP values ranging from 2.26 to 7.17.

**Table 15 - Fragrance Base Composition**

| **Fragrance Base Ingredients** | **Parts bv Weight** | **CAS** |
|---|---|---|
| AGRUNITRILE | 30 | 51566-62-2 |
| ALD AA TRIPLAL TOCO | 30 | 68737-61-1 |
| ALD C-10 TOCO78 | 30 | 112-31-2 |
| ALD C-12 MNA TOCO | 30 | 110-41-8 |
| ALD C-18 | 50 | 104-61-0 |
| BENZ BENZOATE | 210 | 120-51-4 |
| CITRONELLOL 950 | 15 | 94333-77-4 |
| CYCLACET | 70 | 2500-83-6 |
| CYCLAPROP | 120 | 17511-60-3 |
| DAMASCONE DELTA TOCO | 20 | 57378-68-4 |
| Ethyl 2-methyl butyrate | 30 | 7452-79-1 |
| GALBASCONE PRG TOCO | 10 | 56973-85-4 |
| ISO PROPYL MYRISTATE | 30 | 110-27-0 |
| METH IONONE GAMMA A TOCO | 65 | 7388-22-9 |
| PEONILE (ELINCS) | 70 | 10461-98-0 |
| TETRAHYDRO LINALOOL | 90 | 78-69-3 |
| UNDECALACTONE GAMMA COEUR | 100 | 104-67-6 |

Preparation of fabric softener samples containing testing microcapsules: The microcapsule slurry made in accordance with the method described in Example 1 or Example 9 (except the fragrance composition is different as described above) was diluted in water and added to an un-fragranced fabric softener base. The resulting mixture was homogenized by using an overhead stirrer for 15 minutes at 300 rpm. The finished fabric softener samples contained 0.1% neat oil equivalent. Samples were prepared one day prior to the washing experiments.

Stability testing: The finished fabric softener samples were placed at 37 °C for a period of up to 8 weeks. Testing samples were taken out at 4 and 8 weeks respectively for sensory evaluation and fragrance leakage measurements. "Fresh" (Performance) in Table 17 means the finished fabric softener samples were evaluated when they were fresh and before being placed at 37 °C for a prolonged period of time.

Sensory Performance Evaluation: The fragrance intensity of the fragrance composition encapsulated by the testing microcapsule was evaluated by conducting a laundry experiment with the finished fabric softener sample using accepted experimental protocols using European wash machine (Miele). Terry towels were used for the washing experiments and were washed with the finished fabric softener sample containing the testing microcapsule. Washed terry towel samples were removed from the washing machine and line dried overnight. The randomly selected damp towel samples were evaluated by several experts using the intensity scale 0-5, where 0 means "no performance" and 5 means "strong performance". The evaluation was performed "blind", such that each sample had a randomly allocated number. The dry evaluation was performed the day following the damp and was performed by the same experts using the same intensity scale 0-5.

**Table 16 - Evaluation Results**

| **Microcapsule** | **Fragrance Composition** | **FO (%)** | **Leakage 4 weeks 37C** | **Leakage 8 weeks 37C** | **Slurry Color** | **Fabric Softener Color** |
|---|---|---|---|---|---|---|
| polyisocyanate capsule | FB | 0.22 | <10% | <10% | White | White |
| polyisocyanate capsule | FB + 15% Ethyl vanillin | 0.42 | 18% | 16% | White | White |
| polyisocyanate capsule | FB + 2% Benzaldehyde | 0.30 | <10% | <10% | White | White |
| polyisocyanate capsule | FB + 1.5 % Cinnamaldehyde + 1.5% Ethyl vanillin | 0.34 | <10% | <10% | White | White |
| polyisocyanate capsule | FB + 2% Oxyphenylon | 0.23 | Not measured | <10% | White | White |
| polyisocyanate capsule | FB + 7.5 % Oxyphenylon | 0.50 | <10% | <10% | White | White |
| polyisocyanate capsule | FB + 15 % Oxyphenylon | 0.50 | <10% | <10% | White | White |
| polyisocyanate capsule | FB + 1% Veltol | 0.36 | Not measured | <10% | White | White |
| polyisocyanate capsule | FB + 2% Coumarin | 0.31 | <10% | <10% | White | White |
| polyisocyanate capsule | FB + 7.5% Coumarin | 0.50 | 10% | 15% | White | White |
| polyisocyanate capsule | FB + 15% Coumarin | 0.98 | 12% | 17% | White | White |
| polyisocyanate capsule | FB + 2% Aubepine | 0.25 | <10% | <10% | White | White |
| melamine formaldehyde capsule | FB + 15% ethyl vanillin | 4.37 | 74% | 81% | Brown | Brown |

| | | | | | | |
|---|---|---|---|---|---|---|
| Notes: (1) FB means fragrance base. (2) The amount (in percentage) of low logP fragrance ingredient in the fragrance composition is based on the total weight of the fragrance composition. (3) FO means free oil. The amount (in percentage) of free oil in the slurry was measured based on the total weight of the fragrance composition before the slurry was mixed with the fabric softener. (4) "Leakage 4 weeks 37C" means the amount of fragrance leakage from the microcapsule based on the total weight of the fragrance composition, measured after the finished fabric softener samples were placed at 37 °C for a period of 4 weeks. (5) "Leakage 8 weeks 37C" means the amount of fragrance leakage from the microcapsule based on the total weight of the fragrance composition, measured after the finished fabric softener samples were placed at 37 °C for a period of 8 weeks. (6) "Slurry Color" means the color of the microcapsule slurry before the slurry was mixed with the fabric softener. (7) "Fabric Softener Color" is the color of the finished fabric softener sample containing 0.3% neat oil equivalent. | | | | | | |

**Table 17 - Sensory Performance**

| **Microcapsule** | **Fragrance Composition** | **Fresh Performance** | **Fresh Performance Intensity** | **Performance 8 weeks 37C** | **Intensity 8 weeks 37C** |
|---|---|---|---|---|---|
| polyisocyanate capsule | FB | Fragrance base perceived | 2.8 | Fragrance base perceived | 3.0 |
| polyisocyanate capsule | FB + 15% Ethyl vanillin | Clear ethyl vanillin perceived | 4.0 | Clear ethyl vanillin perceived | 3.7 |
| polyisocyanate capsule | FB + 2% Benzaldehyde | Subtle benzaldehyde perceived | 3.0 | Subtle benzaldehyde perceived | 2.8 |
| polyisocyanate capsule | FB + 1.5 % Cinnamaldehyde + 1.5% Ethyl vanillin | Subtle cinnamaldehyde perceived | 3.0 | Subtle cinnamaldehyde perceived | 2.8 |
| polyisocyanate capsule | FB + 2% Oxyphenylon | Clear Oxyphenylon perceived | 3.0 | Clear Oxyphenylon perceived | 3.1 |
| polyisocyanate capsule | FB + 7.5 % Oxyphenylon | Clear Oxyphenylon perceived | 4.0 | Clear Oxyphenylon perceived | 3.5 |
| polyisocyanate capsule | FB + 15 % Oxyphenylon | Clear Oxyphenylon perceived | 4.3 | Clear Oxyphenylon perceived | 3.7 |
| polyisocyanate capsule | FB + 1% Veltol | Clear Veltol perceived | 3.0 | Subtle Veltol perceived | 3.0 |
| polyisocyanate capsule | FB + 2% Coumarin | Clear coumarin perceived | 3.6 | Clear coumarin perceived | 3.3 |
| polyisocyanate capsule | FB + 7.5% Coumarin | Clear coumarin perceived | 3.8 | Clear coumarin perceived | 3.1 |
| polyisocyanate capsule | FB + 15% Coumarin | Clear coumarin perceived | 3.9 | Clear coumarin perceived | 3.1 |
| polyisocyanate capsule | FB + 2% Aubepine | Clear aubepine perceived | 3.2 | Subtle aubepine perceived | 3.1 |
| melamine formaldehyde capsule | FB + 15% ethyl vanillin | Low smell of fragrance | 1.7 | No smell of fragrance | 1.0 |

| | | | | | |
|---|---|---|---|---|---|
| Notes: (1) FB means fragrance base. (2) The amount (in percentage) of low logP fragrance ingredient in the fragrance composition is based on the total weight of the fragrance composition. (3) "Fresh Performance", "Fresh Performance Intensity", "Performance 8 weeks 37C" and "Intensity 8 weeks 37C" all are evaluations of washed terry towel samples after drying and gentle handling. (4) "8 weeks 37C" means after the finished fabric softener samples were placed at 37 °C for a period of 8 weeks. | | | | | |

This Example demonstrated that the core-shell microcapsules of the present disclosure are able to successfully encapsulate fragrance ingredients with low logP (from 0.5 to 2.2). Polyisocyanate microcapsules prepared in accordance with the method described in Example 9 successfully encapsuled fragrances containing 15% ethyl vanillin, 15% oxyphenylon, or 15% coumarin. Such microcapsules demonstrated high encapsulation efficiency (low free oil) for low logP fragrance ingredients, good dry sensory performance and stability. In comparison, melamine formaldehyde microcapsules prepared in accordance with the method described in Example 1 had low encapsulation efficiency (high free oil) for low logP fragrance ingredients, high leakage and poor dry sensory performance. The high leakage of ethyl vanillin caused the slurry turning brown, which would also discolor the commercial product in which the slurry is added. Therefore, the core-shell microcapsules of the present disclosure provide a better delivery system to deliver low logP fragrance and bring a caring, creamy, sweet, oriental, sensorial, luxurious benefit to a consumer product.

Note that not all of the activities described above in the general description or the examples are required, that a portion of a specific activity may not be required, and that one or more further activities may be performed in addition to those described. Still further, the order in which activities are listed are not necessarily the order in which they are performed.

In the foregoing specification, the concepts have been described with reference to specific embodiments. However, one of ordinary skill in the art appreciates that various modifications and changes can be made without departing from the scope of the invention as set forth in the claims below. Accordingly, the specification is to be regarded in an illustrative rather than a restrictive sense, and all such modifications are intended to be included within the scope of invention.

Benefits, other advantages, and solutions to problems have been described above with regard to specific embodiments. However, the benefits, advantages, solutions to problems, and any feature(s) that may cause any benefit, advantage, or solution to occur or become more pronounced are not to be construed as a critical, required, or essential feature of any or all the claims.

It is to be appreciated that certain features are, for clarity, described herein in the context of separate embodiments, may also be provided in combination in a single embodiment. Conversely, various features that are, for brevity, described in the context of a single embodiment, may also be provided separately or in any subcombination.

Every document cited herein, including any cross referenced or related patent or application and any patent application or patent to which this application claims priority or benefit thereof, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

## Claims

1. A core-shell microcapsule slurry comprising:
(a) core-shell microcapsules having diameter of 1 to 100 microns, the core of the microcapsules comprises an active material and the shell of the microcapsules comprises a self-condensed polyisocyanate;
(b) a dispersant comprising denatured pea protein; and
(c) a hydrocolloid comprising gum Arabic;
wherein the active material comprises a low logP fragrance having a logP value ranging from 0.5 to 2.2, the amount of the low logP fragrance is from 2% to 18% by weight based on the weight of the active material, and the core-shell microcapsule slurry is white.

2. The core-shell microcapsule slurry of claim 1, wherein the low logP fragrance is selected from the group consisting of ethyl vanillin, coumarin, 4-(4-hydroxyphenyl)butan-2-one, p-anisaldehyde, 2-ethyl-3-hydroxy-4H-pyran-4-one, benzaldehyde, cinnamaldehyde, and combinations thereof.

3. The core-shell microcapsule slurry of claim 1 or 2, wherein the polyisocyanate is trimethylol propane-adduct of xylylene diisocyanate.

4. The core-shell microcapsule slurry of any one of the preceding claims, further comprising a rheology modifier, a preservative, an emulsifier, or a combination thereof.

5. The core-shell microcapsule slurry of claim 4, wherein the rheology modifier comprises xanthan gum.

6. The core-shell microcapsule slurry of any one of the preceding claims, wherein the self-condensed polyisocyanate is present in an amount of from 0.1% to 8% by weight of the core-shell microcapsule slurry.

7. The core-shell microcapsule slurry of any one of the preceding claims, wherein the active material is a fragrance, a pro-fragrance, a malodor counteractive agent, or a combination thereof.

8. The core-shell microcapsule slurry of claim 7, wherein the active material is a fragrance and the slurry has (a) less than 0.3% or less than 0.25% of a non-encapsulated fragrance, based on total weight of the fragrance in the slurry, (b) a viscosity of less than 600 cps or less than 580 cps as measured at shear rate of 21 s⁻¹, or (c) a combination of (a) and (b).

9. A consumer product comprising the core-shell microcapsule slurry of any one of claims 1 to 8, preferably the consumer product is a fabric conditioner, a fabric softener, a fabric refresher, a liquid laundry detergent, or a powder detergent.

10. A method for producing a core-shell microcapsule slurry of claim 1 comprising:
(a) preparing an aqueous phase by
(i) denaturing a pea protein,
(ii) adjusting the pH of the aqueous phase to below 6, and
(iii) adding gum Arabic as a hydrocolloid to the aqueous phase;
(b) preparing an oil phase comprising an active material and a polyisocyanate;
(c) emulsifying the oil phase into the aqueous phase to form a slurry comprising core-shell microcapsules; and
(d) curing the shell of the microcapsules at a temperature below 80 °C;
wherein the active material comprises a low logP fragrance having a logP value ranging from 0.5 to 2.2, the amount of the low logP fragrance is from 2% to 18% by weight based on the weight of the active material, and the core-shell microcapsule slurry is white.

11. The method of claim 10, wherein the polyisocyanate is trimethylol propane-adduct of xylylene diisocyanate.

12. The method of claim 10 or 11, wherein the pH in (a)(ii) is adjusted to between 4.5 and 3.5.

13. The method of any one of claims 10 to 12, wherein the shell of the microcapsules in (d) is cured at a temperature of from 63 °C to 67 °C.

14. The method of any one of claims 10 to 13, wherein the active material is a fragrance, a pro-fragrance, a malodor counteractive agent, or a combination thereof.

15. The method of claim 14, wherein the active material is a fragrance and the slurry has (a) less than 0.3% or less than 0.25% of a non-encapsulated fragrance, based on total weight of the fragrance, (b) a viscosity of less than 600 cps or less than 580 cps as measured at shear rate of 21 s⁻¹, or (c) a combination of (a) and (b).

16. The method of any one of claims 10 to 15, further comprising adding a rheology modifier, a preservative, an emulsifier, or a combination thereof.

17. The method of claim 16, wherein the rheology modifier is added prior to step (c), preferably the rheology modifier is xanthan gum.

18. The method of any one of claims 10 to 17, wherein the amount of the polyisocyanate is from 0.1% to 8% by weight of the core-shell microcapsule slurry.
